(19) Europäisches Patentamt
European Patent Office
Office européen des brevets

(11) Veröffentlichungsnummer: **0 450 420 B1**

# (12) EUROPÄISCHE PATENTSCHRIFT

(45) Veröffentlichungstag der Patentschrift: **21.09.94**

(21) Anmeldenummer: **91104484.0**

(22) Anmeldetag: **22.03.91**

(51) Int. Cl.[5]: **C07D 413/04**, C07D 417/04, C07D 491/04, C07D 263/56, C07D 277/66, C07D 413/14, C07D 417/14, A61K 31/44, //(C07D491/04,307:00,221:00)

## (54) Heterocyclisch substituierte Dihydropyridine, Verfahren zu ihrer Herstellung und ihre Verwendung in Arzneimitteln.

(30) Priorität: **06.04.90 DE 4011106**

(43) Veröffentlichungstag der Anmeldung:
**09.10.91 Patentblatt 91/41**

(45) Bekanntmachung des Hinweises auf die Patenterteilung:
**21.09.94 Patentblatt 94/38**

(84) Benannte Vertragsstaaten:
**AT BE CH DE DK ES FR GB GR IT LI LU NL SE**

(56) Entgegenhaltungen:
**EP-A- 0 080 220**
**EP-A- 0 217 530**

(73) Patentinhaber: **BAYER AG**

**D-51368 Leverkusen (DE)**

(72) Erfinder: **Goldmann, Siegfried, Dr.**
**Am Osterholz 91**
**W-5600 Wuppertal 11 (DE)**

Erfinder: **Böshagen, Horst, Dr.**
**Wiesenstrasse 4**
**W-5657 Haan (DE)**
Erfinder: **Stoltefuss, Jürgen, D.I.**
**Parkstrasse 20**
**W-5657 Haan (DE)**
Erfinder: **Straub, Alexander, Dr.**
**Moospfad 30**
**W-5600 Wuppertal 1 (DE)**
Erfinder: **Gross, Reiner, Prof. Dr.**
**Platzhofstrasse 23**
**W-5600 Wuppertal 1 (DE)**
Erfinder: **Hütter, Joachim, Dr.**
**Weinhaeuserstrasse 9**
**W-5090 Leverkusen (DE)**
Erfinder: **Hebisch, Siegbert, Dr.**
**Richard-Seel-Weg 11**
**W-5600 Wuppertal 1 (DE)**
Erfinder: **Bechem, Martin, Dr.**
**Hans-Boeckler-Strasse 102**
**W-5600 Wuppertal 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 450 420 B1

## Beschreibung

Die Erfindung betrifft neue heterocyclisch substituierte Dihydropyridine, Verfahren zu ihrer Herstellung und ihre Verwendung in Arzneimitteln, insbesondere in Mitteln mit positiv inotroper Wirkung.

Es ist bereits bekannt, daß 1,4-Dihydropyridine gefäßerweiternde Eigenschaften besitzen und als Coronarmittel und Antihypertensiva verwendet werden können [vgl. Brit. Patent 1 173 062 und 1 358 951; DE-OS 2 629 892 und 27 52 820]. Weiterhin ist bekannt, daß 1,4-Dihydropyridine eine Hemmung der Kontraktionskraft von glatten und cardialen Muskeln bewirken und zur Behandlung von Coronar- und Gefäßerkrankungen eingesetzt werden können [vgl. Fleckenstein, Ann. Rev. Pharmacol. Toxicol., 17, 149 - 166 (1977)].

Es ist außerdem bekannt, daß 3-Nitro-dihydropyridine im allgemeinen neben einer positiv inotropen Herzwirkung den Nachteil einer unerwünschten konstringierenden Wirkung an den Koronargefäßen zeigen können [vgl. Schramm et al., Nature 303, 535-537 (1983) und DE-OS 3 447 169].

Bei Kenntnis des Standes der Technik war es nicht vorhersehbar, daß die erfindungsgemäßen Verbindungen eine kontraktionskraftverstärkende, am Herzmuskel positiv inotrope Wirkung mit weitgehend gefäßneutralem Verhalten besitzen.

Die Erfindung betrifft neue heterocyclisch substituierte Dihydropyridine der allgemeinen Formel (I),

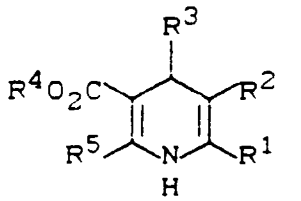

(I)

in welcher

$R^1$ und $R^5$ gleich oder verschieden sind und für geradkettiges oder verzweigtes Alkyl mit bis zu 8 Kohlenstoffatomen stehen,

$R^2$ für Nitro oder Cyano steht,

oder

$R^1$ und $R^2$ gemeinsam einen Lactonring der Formel

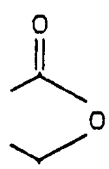

bilden,

$R^3$ für einen Rest der Formel

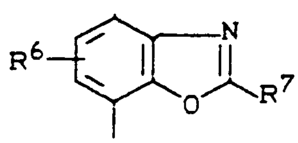

,

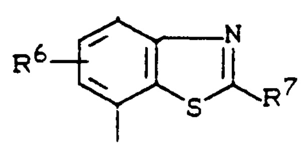

,

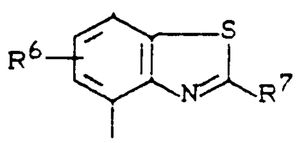

oder

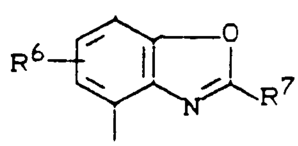

steht,
worin

$R^6$
- Wasserstoff, Halogen oder geradkettiges oder verzweigtes Alkyl oder Alkoxy mit jeweils bis zu 8 Kohlenstoffatomen bedeutet,

$R^7$
- Aryl mit 6 bis 10 Kohlenstoffatomen bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder
- Thienyl oder Pyridyl bedeutet,

$R^4$ für Wasserstoff steht, oder
für geradkettiges oder verzweigtes Alkyl, Alkenyl, Alkadienyl oder Alkinyl mit jeweils bis zu 10 Kohlenstoffatomen steht, die gegebenenfalls bis zu 2-fach gleich oder verschieden durch Halogen, Hydroxy, Carboxy, Cyano, Nitro, Phenoxy oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können

und deren physiologisch unbedenklichen Salze.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher

$R^1$ und $R^5$ gleich oder verschieden sind und
für geradkettiges oder verzweigtes Alkyl mit bis zu 6 Kohlenstoffatomen stehen,

$R^2$ für Nitro oder Cyano steht,

oder

$R^1$ und $R^2$ gemeinsam einen Lactonring der Formel

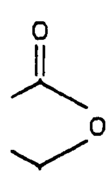

bilden,

$R^3$ für einen Rest der Formel

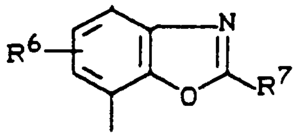 , 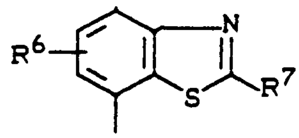 ,

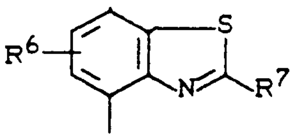 oder 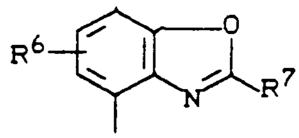

steht,
worin

$R^6$
- Wasserstoff, Fluor, Chlor oder geradkettiges oder verzweigtes Alkyl oder Alkoxy mit jeweils bis zu 2 Kohlenstoffatomen bedeutet,

$R^7$
- Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist, oder
- Pyridyl oder Thienyl bedeutet,

$R^4$
- für Wasserstoff oder für geradkettiges oder verzweigtes Alkyl oder Alkenyl mit jeweils bis zu 8 Kohlenstoffatomen steht, die gegebenenfalls durch Fluor, Chlor, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 6 Kohlenstoffatomen oder durch Phenoxy oder Phenyl substituiert sind

und deren physiologisch unbedenklichen Salze.

Besonders bevorzugt sind Verbindungen der allgemeinen Formel (I), in welcher

$R^1$ und $R^5$ gleich oder verschieden sind und
- für geradkettiges oder verzweigtes Alkyl mit bis zu 4 Kohlenstoffatomen stehen,

$R^2$ für Nitro oder Cyano steht,

oder

$R^1$ und $R^2$ gemeinsam einen Lactonring der Formel

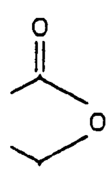

bilden,

$R^3$ für einen Rest der Formel

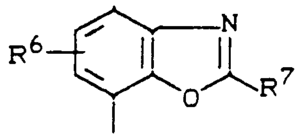 , 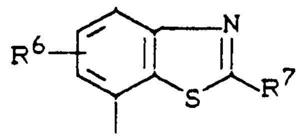 ,

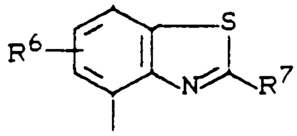 oder 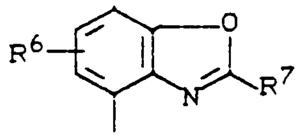

steht,
worin

$R^6$
- Wasserstoff, Chlor oder Methyl bedeutet,

$R^7$
- Phenyl bedeutet, das gegebenenfalls durch Fluor, Chlor, Nitro, Trifluormethyl oder
- geradkettiges oder verzweigtes Alkyl, Acyl oder Alkoxy mit jeweils bis zu 4 Kohlenstoffatomen substituiert ist, oder
- Pyridyl bedeutet

$R^4$
- für Wasserstoff steht, oder
- für geradkettiges oder verzweigtes Alkyl mit bis zu 6 Kohlenstoffatomen steht, das gegebenenfalls durch Hydroxy, Carboxy, Cyano oder durch geradkettiges oder verzweigtes Alkoxycarbonyl, Alkoxy oder Acyloxy mit jeweils bis zu 4 Kohlenstoffatomen substituiert ist

und deren physiologisch unbedenklichen Salze.

Außerdem wurde ein Verfahren zur Herstellung der erfindungsgemäßen Verbindungen der allgemeinen Formel (I), gefunden, dadurch gekennzeichnet,
daß man im Fall, daß $R^1$ und $R^2$ die oben angegebene Bedeutung haben, aber nicht gemeinsam einen Lactonring bilden,

[A] Verbindungen der allgemeinen Formel (II)

$R^3$-CHO (II)

in welcher
$R^3$ die oben angegebene Bedeutung hat,
zunächst mit Acetessigestern der allgemeinen Formel (III)

$R^5$-CO-$CH_2$-$CO_2$-$R^4$ (III)

in welcher
$R^4$ und $R^5$ die oben angegebene Bedeutung haben,
gegebenenfalls unter Isolierung der entsprechenden Ylidenverbindungen der allgemeinen Formel (IV)

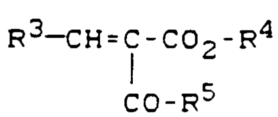 (IV)

in welcher
$R^3$, $R^4$ und $R^5$ die oben angegebene Bedeutung haben,
umsetzt und anschließend
entweder mit Verbindungen der Formel (V)

$R^1$-CO-$CH_2$-$R^2$ (V)

in welcher

$R^1$ und $R^2$ die oben angegebene Bedeutung haben,

in inerten Lösemitteln, in Anwesenheit von Ammoniak oder Ammoniumsalzen, oder direkt mit Enaminoderivaten der allgemeinen Formel (VI)

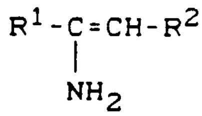

(VI)

in welcher

$R^1$ und $R^2$ die oben angegebene Bedeutung haben,

umsetzt,

oder

[B] die Aldehyde der allgemeinen Formel (II) zunächst mit den Verbindungen der allgemeinen Formel (V), gegebenenfalls unter Isolierung der Ylidenverbindungen der allgemeinen Formel (VII)

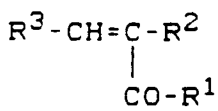

(VII)

in welcher

$R^1$, $R^2$ und $R^3$ die oben angegebene Bedeutung haben,

umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII)

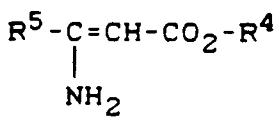

(VIII)

in welcher

$R^4$ und $R^5$ die oben angegebene Bedeutung haben,

umsetzt,

oder im Fall, daß $R^1$ und $R^2$ gemeinsam einen Lactonring bilden,

[C] zunächst nach denen unter [A] und [B] aufgeführten Methoden, Verbindungen der allgemeinen Formel (Ia)

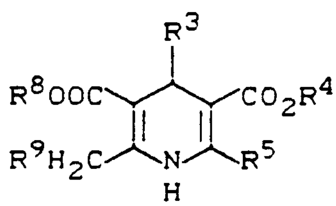

(Ia)

in welcher

$R^3$, $R^4$ und $R^5$ die oben angegebene Bedeutung haben,

$R^8$ für einen $C_1$-$C_6$-Alkyl-Rest steht

und

$R^9$ für eine Abgangsgruppe wie beispielsweise Chlor oder Acetoxy steht,

herstellt und nach bekannten Verfahren einen säure- oder basenkatalysierten Ringschluß anschließt,

und im Fall, daß $R^4$ nicht Wasserstoff bedeutet,

[D] Verbindungen der allgemeinen Formel (I), in welcher $R^1$, $R^2$, $R^3$, $R^4$ und $R^5$ die oben angegebene Bedeutung haben und $R^4$ für Wasserstoff steht, gegebenenfalls über ein reaktives Säurederivat, mit den entsprechenden Alkoholen umsetzt, wobei durch Einsatz der enantiomerenreinen Carbonsäure die entsprechenden Enantiomere der Ester erhalten werden.

Die erfindungsgemäßen Verfahren können durch folgendes Formelschema erläutert werden:

[A]

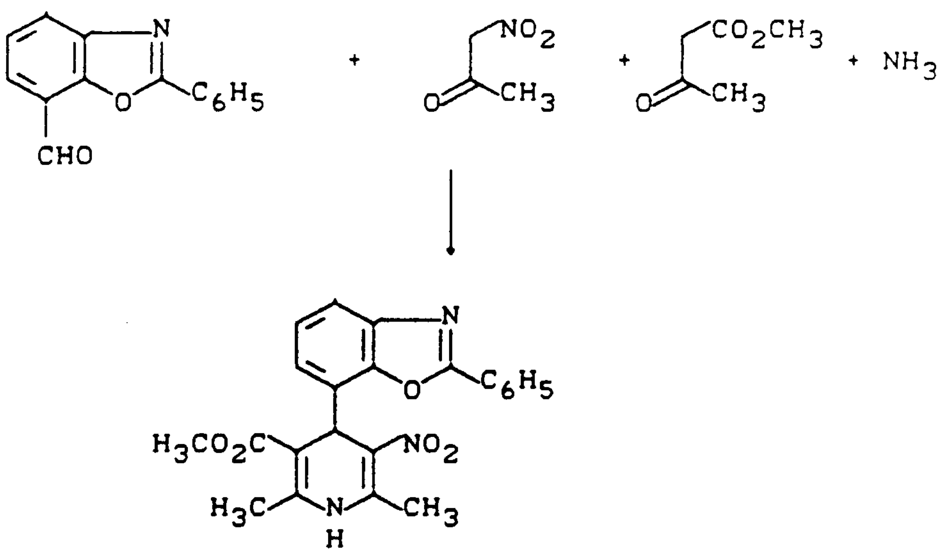

[A]

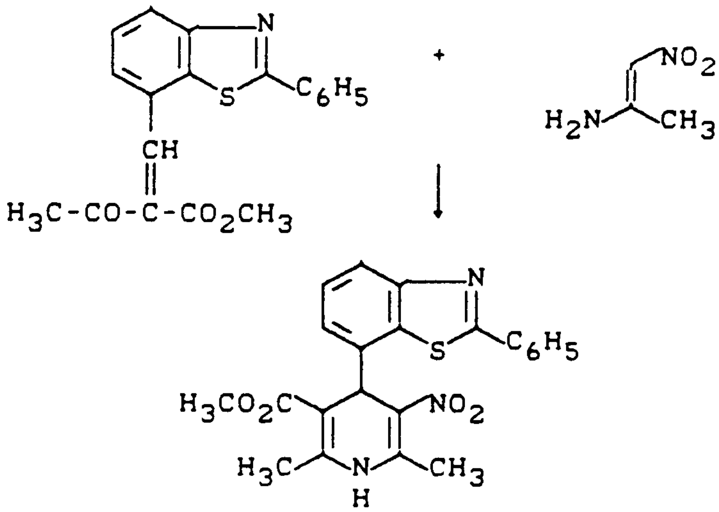

[B]

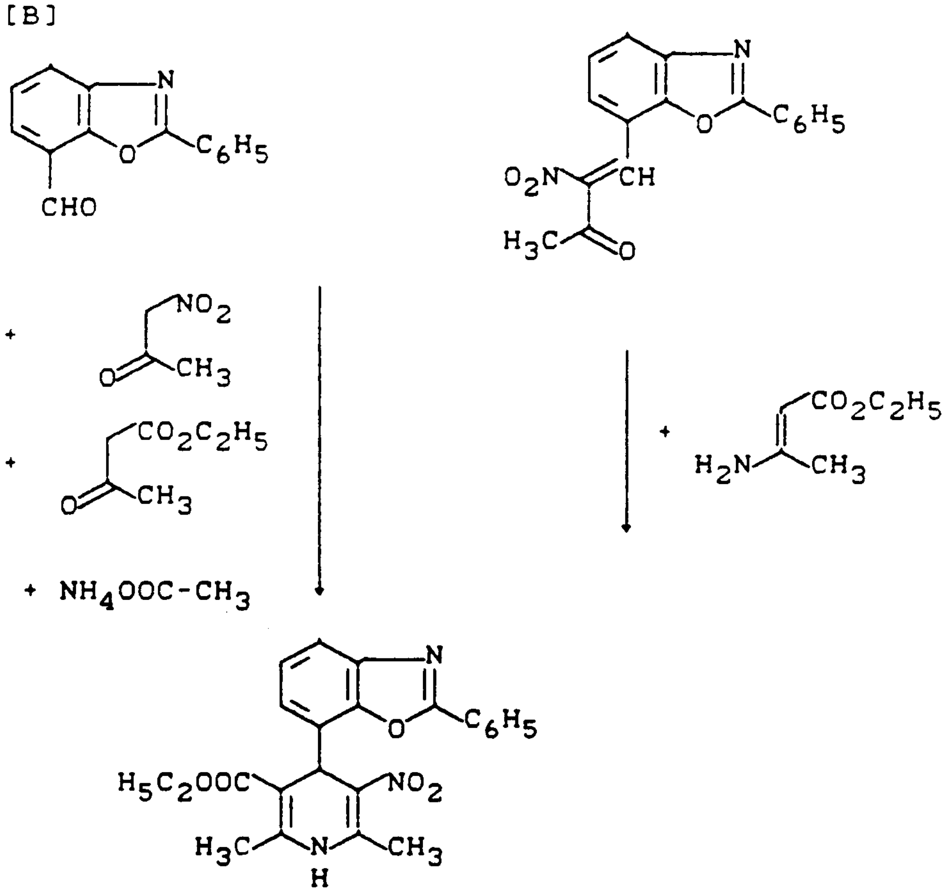

[C]

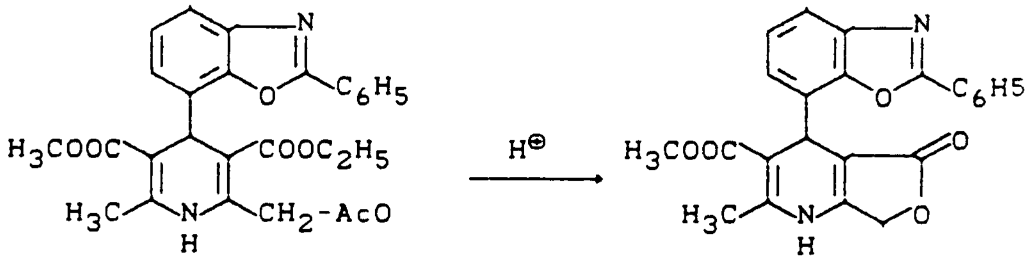


[D]

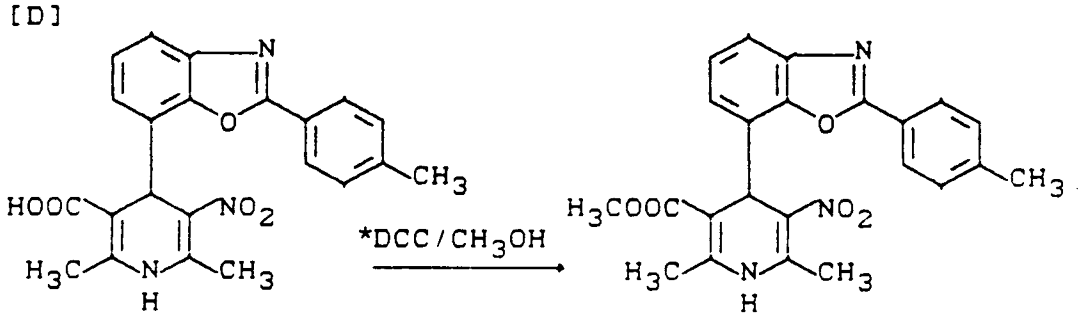


* = Dicyclohexylcarbodiimid

Als Lösemittel eignen sich für die Verfahren [A], [B] und [C] alle inerten organischen Lösemittel. Hierzu gehören bevorzugt Alkohole wie Methanol, Ethanol, n- bzw. iso-Propanol, Ether wie Diethylether, Tetrahydrofuran, Dioxan oder Glykolmono- oder diethylether, Eisessig, Pyridin, Dimethylformamid, Dimethylsulfoxid, Acetonitril oder Hexamethylphosphorsäuretriamid oder Toluol.

Als Lösemittel für das Verfahren [D] eignen sich die oben aufgeführten Lösemittel mit Ausnahme der Alkohole.

Die Reaktionstemperatur für die Verfahren [A], [B], [C] und [D] können in einem größeren Bereich variiert werden, Im allgemeinen arbeitet man in einem Bereich von 10° C bis 200° C, bevorzugt von 20° C bis 150° C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B., indem man Diastereomerengemische der Verbindungen der allgemeinen Formel (I), in welcher $R^4$ für einen optischen Esterrest steht, nach üblicher Methode trennt, anschließend die enantiomerenreinen Carbonsäuren herstellt und dann gegebenenfalls durch Veresterung mit entsprechenden Alkoholen in die enantiomerenreinen Dihydropyridine überführt,

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Veresterung der enantiomerenreinen Dihydropyridine erfolgt bevorzugt in Ethern wie Diethylether oder Tetrahydrofuran, Dimethylformamid, Methylenchlorid, Chloroform, Acetonitril oder Toluol.

Die Aldehyde der allgemeinen Formel (II) sind ebenfalls neu und können hergestellt werden, indem man

a) entweder Verbindungen der allgemeinen Formel (IX)

$R^{3'}$-$CH_3$ (IX)

in welcher

$R^{3'}$ für die Reste der Formel

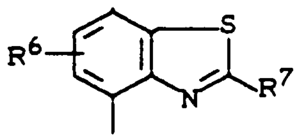

oder

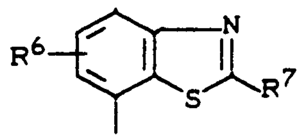

steht,

in welcher

$R^6$ und $R^7$ die oben angegebene Bedeutung haben,

zu Verbindungen der allgemeinen Formel (X)

$R^{3'}$-$CH_2$-Hal (X)

in welcher

$R^{3'}$ die oben angegebene Bedeutung hat

und

Hal für Halogen, vorzugsweise für Brom steht,

halogeniert, mit Acetationen umsetzt und anschließend zu Verbindungen der allgemeinen Formel (XI)

$R^{3'}$-$CH_2OH$ (XI)

in welcher

$R^{3'}$ die oben angegebene Bedeutung hat

verseift und in einem letzten Schritt nach üblicher Methode oxidiert,

b) oder indem man Verbindungen der allgemeinen Formel (XII)

$R^{3''}$-$CH_3$ (XII)

in welcher

$R^{3''}$ für einen Rest der Formel

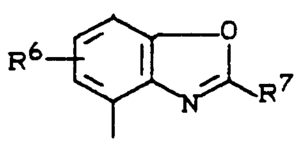

oder

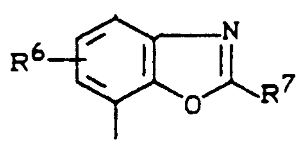

steht,

in welcher

$R^6$ und $R^7$ die oben angegebene Bedeutung haben,

zu Verbindungen der allgemeinen Formel (XIII)

$R^{3''}$-$CH(Br)_2$ (XIII)

in welcher

$R^{3''}$ die oben angegebene Bedeutung hat

halogeniert und anschließend nach üblicher Methode hydrolysiert.

Das erfindungsgemäße Verfahren kann durch folgendes Formelschema beispielhaft erläutert werden:

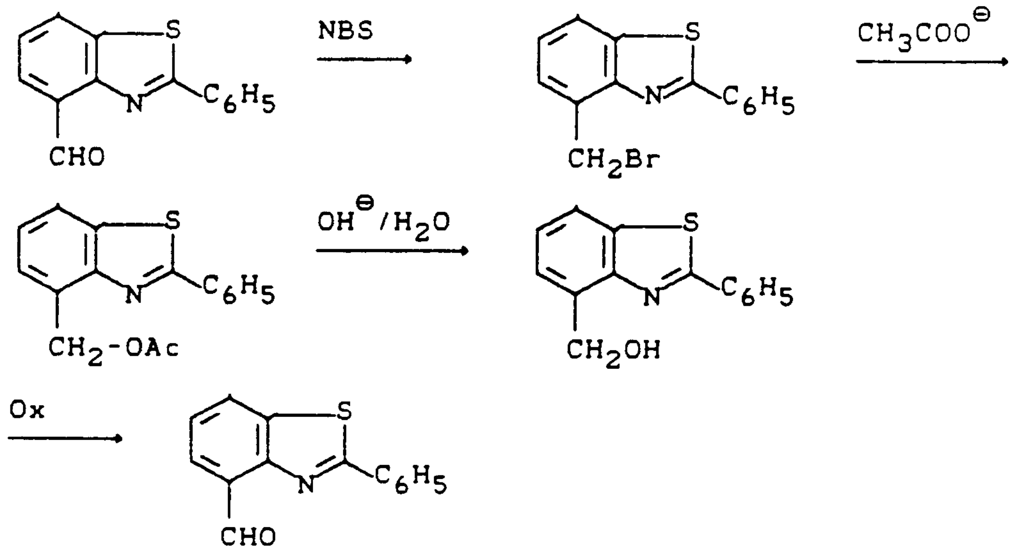


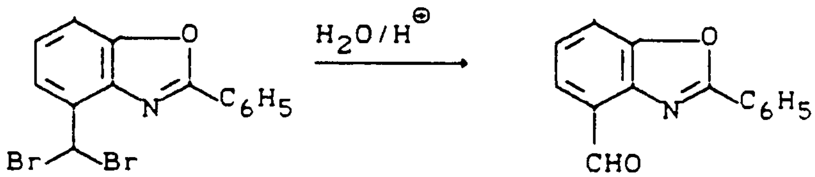


Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Als Oxidationsmittel eignen sich im Fall der Hydroxymethyl-Verbindungen beispielsweise Mangandioxid, Dimethylsulfoxid, Cer-Ammoniumnitrat, Dipyridin-Chrom(VI)oxid, Natriumdichromat, Jodosobenzol, Pyridinchlorochromat, Silbercarbonat auf Celit oder Jones-Reagenz. Bevorzugt ist Mangandioxid.

Die Oxidationen und Reduktionen können bei Normaldruck oder erhöhtem oder erniedrigtem Druck (beispielsweise von 0,5 bis 5 bar), vorzugsweise bei Normaldruck durchgeführt werden.

Die Verbindungen der allgemeinen Formel (IX) sind bekannt [vgl. Acta Chem. Second Ser B., 31, 203 (1977)].

Die Verbindungen der allgemeinen Formel (X) sind bekannt oder können nach literaturbekannten Methoden hergestellt werden [vgl. J. Org. Chem. 46, 3259 (1981)].

Die Acetessigester der Formel (III) sind bekannt oder können nach üblichen Methoden hergestellt werden [vgl. D. Borrmann, "Umsetzung von Diketonen mit Alkoholen, Phenolen und Mercaptanen", in Houben-Weyl, Methoden der organischen Chemie, Vol. VIII/4, 230 ff. (1968)].

Die Ylidenverbindungen (IV) und (VII) sind neu, können aber nach üblichen Methoden hergestellt werden [vgl. H. Dornow und W. Sassenberg, Liebigs Ann. Chem. 602, 14 (1957)].

Die Aminocrotonsäurederivate der Formel (VI) und (VIII) sind bekannt oder können nach bekannten Methoden hergestellt werden [S.A. Glickman, A.C. Cope, J. Am. Chem. Soc. 67, 1017 (1946)].

Die Verbindungen der allgemeinen Formel (V) sind ebenfalls bekannt [vlg. N. Levy, C.W. Scaife, J. Chem. Soc. (London) 1946, 1100; C.D. Hurd, M.E. Nilson, J. Org. Chem. 20, 927 (1955)].

Die vorstehenden Herstellungsverfahren sind lediglich zur Verdeutlichung angegeben. Die Herstellung der Verbindungen der Formel (I) und (II) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Sie können deshalb in Arzneimitteln zur Beeinflussung des pathologisch veränderten Blutdrucks, als

Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden.

Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (1) (118,5 mmol/l NaCl, 4.75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32° C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965), 529 - 541). Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdrucks eine Koronardilatation, eine Zu- bzw, Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellung der Ausgangsverbindungen

Beispiel I

4-Brommethyl-2-phenyl-benzthiazol

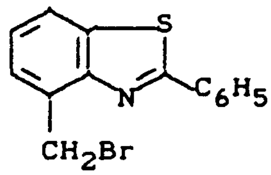

42,8 g (190 mmol) 4-Methyl-2-phenyl-benzthiazol [vgl. Perregaard, Lawesson, Acta Chem. Scand. Ser, B, 31, 203 (1977)] wurden in 1 l Tetrachlorkohlenstoff gelöst, mit katalytischen Mengen 2,2'-Azoisobuttersäurenitril versetzt, Unter Rückfluß wurden 40,6 g (0,228 mol) N-Bromsuccinimid portionsweise zugegeben und über Nacht gekocht. Es wurden weitere 40,6 g NBS zugegeben und 2h gekocht. Nach Abkühlung wurde mit Methylenchlorid verdünnt, mit Wasser gewaschen, getrocknet, eingeengt und der Rückstand mit Petrolether kristallisiert.
Ausbeute: 53 g (92% der Theorie)
Fp.: 114-115° C

Beispiel II

4-Acetoxymethyl-2-phenyl-benzthiazol

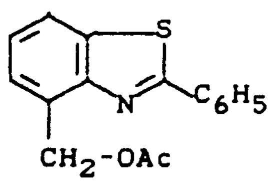

53 g (0,176 mol) der Verbindung aus Beispiel 1 und 20 g (0,2 mol) Kaliumacetat wurden in 500 ml Dimethylformamid 24 h bei Raumtemperatur gerührt, im Vakuum abgezogen, mit Wasser versetzt, abgesaugt und getrocknet.
Ausbeute: 46 g (92% der Theorie)
Fp.: 83-84° C

Beispiel III

4-Hydroxy-2-phenyl-benzthiazol

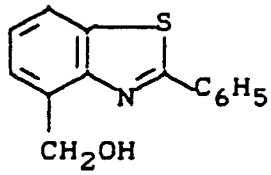

43 g der Verbindung aus Beispiel II wurden in 1 l absolutem Methanol gelöst, mit 5 g Kaliumcarbonat versetzt und nach 10 Minuten bei Raumtemperatur eingeengt. Es wurde mit Methylenchlorid aufgenommen,

gewaschen, getrocknet und eingeengt. Man erhielt 90% der Theorie der Titelverbindung.
Fp.: 116-117° C

Beispiel IV

2-Phenyl-benzthiazol-4-carbaldehyd

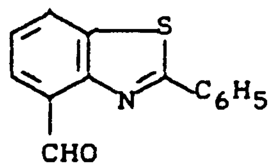

26,5 g (0,11mol) der Verbindung aus Beispiel III wurden in 400 ml Methylenchlorid gelöst und mit 50 g Mangandioxid (aktiv) über Nacht am Rückfluß gekocht. Nach Abkühlung wurde abgesaugt und eingeengt.
Ausbeute: 25 g (85% der Theorie)
Fp.: 127-128° C

Beispiel V

2'-Chlor-3'-methyl-benzanilid

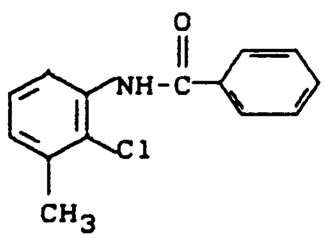

57 g (0,4 mol) 2-Chlor-3-methyl-anilin [CA 89, 30772h) wurden in 1,5 l Toluol gelöst, mit 55 g Kaliumcarbonat versetzt und bei 80° C tropfenweise mit 62 g (0,4 mol) Benzoylchlorid versetzt. Nach vollständiger Umsetzung wurde kalt abfiltriert, mit Wasser gewaschen und getrocknet. Nach Einengen und Verreiben mit Petrolether kristallisierten 98 g (100%).
Fp.: 125-126° C

Beispiel VI

2'-Chlor-3'-methyl-thiobenzanilid

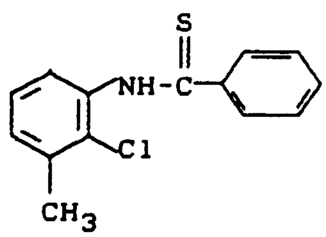

98 g (0,4 mol) der Verbindung aus Beispiel V und 162 g (0,4 mol) Lawessons Reagenz wurden in 1 l Toluol über Nacht gekocht, eingeengt und über $SiO_2$ gereinigt (Cyclohexan/Essigester = 8:2)

Ausbeute: 93 g (80% der Theorie) als gelbes Öl.

Beispiel VII

7-Methyl-2-phenyl-benzthiazol

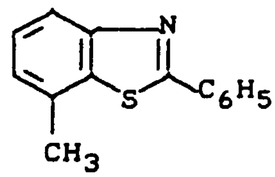

91,6 g (0,35 mol) der Verbindung aus Beispiel VI wurden in 1 l Dimethylformamid mit 64 g (0,42 mol) Diazabicycloundecan 3 h am Rückfluß gekocht. Es wurde eingeengt, in Methylenchlorid/Wasser aufgenommen, schwach sauer gestellt, mit Wasser gewaschen, getrocknet und eingeengt.
Ausbeute: 70 g (89% der Theorie)
Fp.: 53-54° C

Beispiel VIII

7-Brommethyl-2-phenyl-benzthiazol

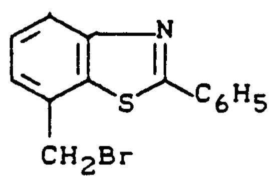

Die Verbindung wurde in Analogie zu Beispiel I erhalten.
Fp.: 104-105° C

Beispiel IX

7-Acetoxymethyl-2-phenyl-benzthiazol

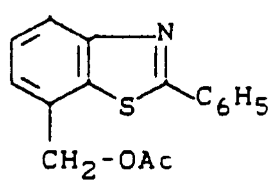

Die Verbindung wurde in Analogie zur Vorschrift für Beispiel II erhalten.
Fp.: 64-65° C

Beispiel X

7-Hydroxymethyl-2-phenyl-benzthiazol

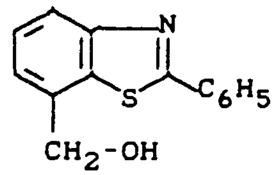

Die Verbindung wurde in Analogie zur Vorschrift für Beispiel III erhalten.
Fp.: 86-87° C

Beispiel XI

2-Phenyl-benzthiazol-7-carbaldehyd

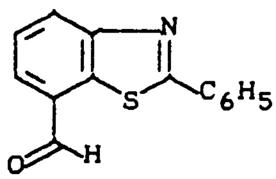

Die Verbindung wurde in Analogie zur Vorschrift für Beispiel IV erhalten.
Fp.: 136° C

Beispiel XII

4-Dibrommethyl-2-phenyl-benzoxazol

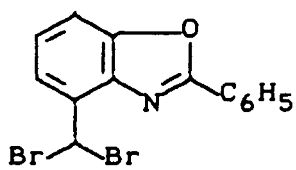

3,5 g (17 mmol) 4-Methyl-2-phenyl-benzoxazol [J. Org. Chem. 46, 3259 (1981)] und 6 g N-Bromsuccinimid wurden in 150 ml Tetrachlorkohlenstoff mit katalytischen Mengen 2,2'-Azoisobuttersäurenitril am Rückfluß gekocht. Nach 7 Stunden wurden weitere 6 g N-Bromsuccinimid und 2,2'-Azoisobuttersäurenitril zugesetzt und weitere 7 h gekocht. Nach Abkühlung wurde mit Wasser gewaschen, getrocknet und eingeengt.
Ausbeute: 5,9 g (96% der Theorie)
Fp.: 106° C

Beispiel XIII

2-Phenylbenzoxazol-4-carbaldehyd

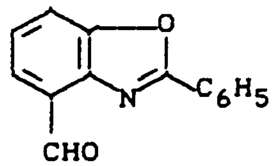

5,5 g (14,9 mmol) der Verbindung aus Beispiel XII wurden mit 30 ml konzentrierter Schwefelsäure 1 h bei 100° C gerührt, auf Wasser gegossen, abgesaugt und getrocknet.
Ausbeute: 3,3 g (100% der Theorie)
Fp.: 116° C

Beispiel XIV:

3-Benzoylamino-2-hydroxy-benzoesäuremethylester

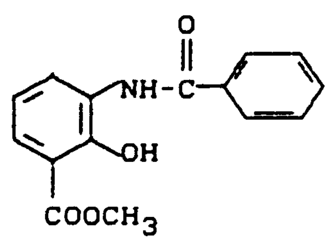

20 mmol 3-Amino-2-hydroxy-benzoesäuremethylester wurden mit 20 mmol Benzoylchlorid unter Zusatz von Dimethylanilin in Toluol 4 Stunden bei Raumtemperatur gerührt, eingeengt und aus EtOH umkristallisiert.
FP: 105° C.

Beispiel XV:

2-Phenyl-benzoxazol-7-carbonsäuremethylester

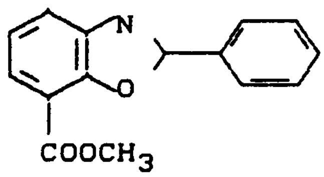

30 mmol der Verbindung aus Beispiel XIV, 30 mmol Benzoesäure, 50 mmol Triphenylphosphin und 120 mmol Triethylamin wurden in 100 ml Acetonitril bei 3° C unter Argon innerhalb von 15 min mit 5,8 ml $CCl_4$ in 5 ml Acetonitril versetzt und 20 Stunden bei Raumtemperatur gerührt, eingeengt, Rückstand mit Diethylether versetzt, abgesaugt, Filtrat eingeengt und an Kieselgel getrennt. Fp: 77° C (weiße Nadeln)

Beispiel XVI:

7-Hydroxymethyl-2-phenyl-benzoxazol

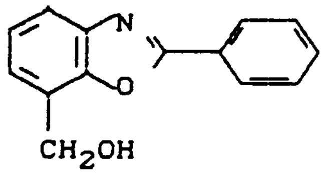

10 mmol der Verbindung aus Beispiel XV wurden in 50 ml Toluol gelöst, dann bei 0° C mit 40 ml DIBAH*(20%ig in Toluol) versetzt und 2 h bei Raumtemperatur gerührt. Nach Aufarbeitung mit Schwefelsäure erhält man die Titelverbindung. Fp: 131° C (aus Ether)

Beispiel XVII:

2-Phenyl-benzoxazol-7-carbaldehyd

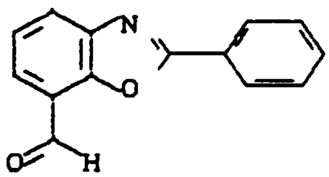

10 mmol der Verbindung aus Beispiel XVI wurden mit 50 mmol $MnO_2$ (aktiv) in 50 ml Aceton 6 h am Rückfluß gekocht, abgesaugt und eingeengt. Der Rückstand wurde aus Isopropanol umkristallisiert. Fp: 107° C

Beispiel XVIII:

2-(3-Pyridyl)-benzoxazol-7-carbaldehyd

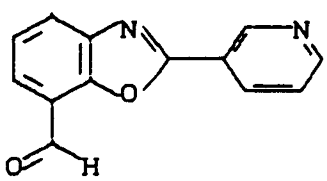

Die Herstellung erfolgte analog der Vorschriften der Beispiele XIV bis XVII.
Fp: 140° C (gelben Nadeln).

* = Diisobutylaluminiumhydrid

Herstellungsbeispiele

Beispiel 1

3-Cyano-2,6-dimethyl-4-(2-phenyl-benzthiazol-7-yl)-1,4-dihydropyridin-5-carbonsäure-isopropylester

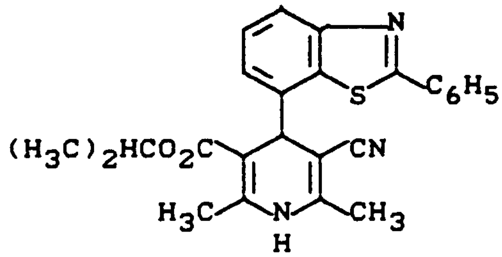

10 mmol 2-Phenyl-benzthiazol-7-carboxaldehyd, 10 mmol Acetessigsäureisopropylester und 10 mmol 3-Aminocrotonsäurenitril werden in 20 ml Ethanol über Nacht gekocht, eingeengt und an Kieselgel gesäult (Cyclohexan/Essigester = 6:4)
Ausbeute: 28% der Theorie
Fp.: 218° C
Die in den Tabellen 1, 2 und 3 aufgeführten Beispiele wurden in Analogie zur Vorschrift des Beispiels 1 hergestellt:

Tabelle 1

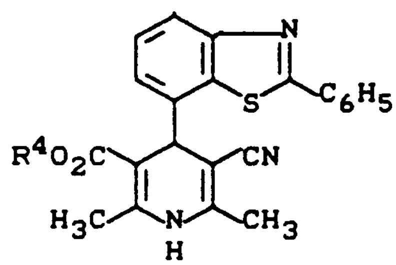

| Beispiel-Nr. | $R^4$ | F (°C) |
|---|---|---|
| 2 | $-C_2H_5$ | 249 |
| 3 | $-CH_3$ | 240-241 |
| 4 | $-CH(CH_3)-CO_2C_2H_5$ | 178 |
| 5 | $-CH(CH_3)-CO_2H$ | amorph |

Tabelle 2

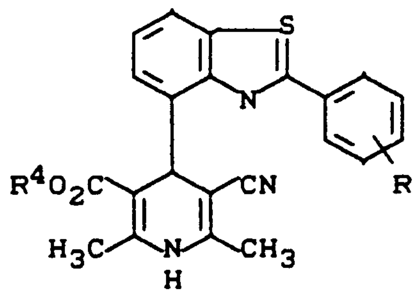

| Beispiel-Nr. | $R^4$ | R | F (°C) |
|---|---|---|---|
| 6 | $-CH_3$ | H | 218 |
| 7 | $-C_2H_5$ | H | 236 |
| 8 | $-CH(CH_3)_2$ | H | 198 |
| 9 | $-C_2H_5$ | 2-Cl | 241 |
| 10 | $-C_2H_5$ | 3-F | 222 |
| 11 | $-C_2H_5$ | 4-F | 254 |
| 12 | $-C_2H_5$ | 2-F | 216 |
| 13 | $-C_2H_5$ | 3-Cl | 232 |
| 14 | $-C_2H_5$ | 4-Cl | 212 |

Tabelle 3

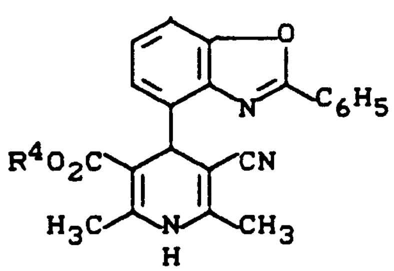

| Beispiel-Nr. | $R^4$ | F (°C) |
|---|---|---|
| 15 | $-C_2H_5$ | 246 |

Beispiel 16

2,6-Dimethyl-3-nitro-4-(2-phenyl-benzthiazol-7-yl)-1,4-dihydropyridin-5-carbonsäuremethylester

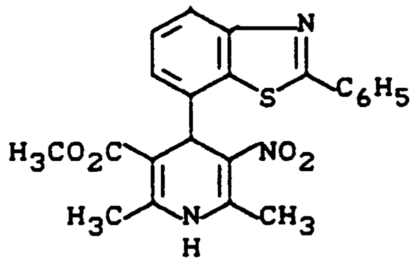

10 mmol 2-Phenyl-benzthiazol-7-carboxaldehyd, 10 mmol Nitroaceton und 10 mmol 3-Aminocrotonsäuremethylester werden in 20 ml Ethanol über Nacht gekocht, eingeengt und an Kieselgel gesäult (Cyclohexan/Essigester = 6:4)
Ausbeute: 25% gelbe Kristalle
Fp.: 233° C (Zers.)
Die in den Tabellen 4, 5 und 6 aufgeführten Beispiele wurden in Analogie zur Vorschrift des Beispiels 17 hergestellt:

Tabelle 4

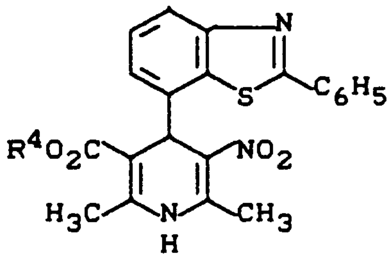

| Beispiel-Nr. | $R^4$ | F (°C) |
|---|---|---|
| 17 | $-C_2H_5$ | 222 |
| 18 | $-CH(CH_3)_2$ | 215 |

Tabelle 5

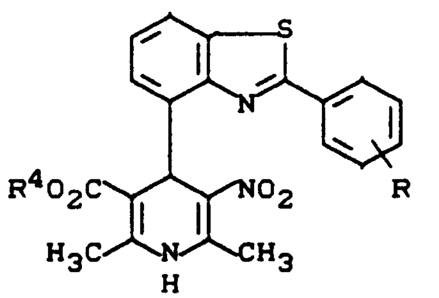

| Beispiel-Nr. | $R^4$ | R | F (°C) |
|---|---|---|---|
| 19 | $-C_2H_5$ | H | 234 |
| 20 | $-C_2H_5$ | 2-Cl | 196 |

Fortsetzung Tabelle 5

| Beispiel-Nr. | $R^4$ | R | F (°C) |
|---|---|---|---|
| 21 | $-C_2H_5$ | 3-F | 204 |
| 22 | $-C_2H_5$ | 4-F | 238 |
| 23 | $-C_2H_5$ | 2-F | 221 |
| 24 | $-C_2H_5$ | 3-Cl | 221 |
| 25 | $-C_2H_5$ | 4-Cl | 246 |

Tabelle 6

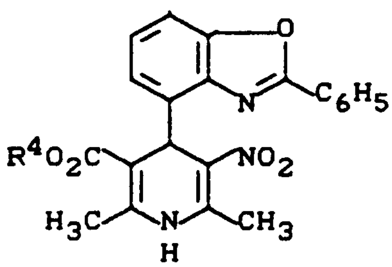

| Beispiel-Nr. | $R^4$ | F (°C) |
|---|---|---|
| 26 | $-C_2H_5$ | 229 |

Beispiel 27

2-Methyl-5-oxo-4-(2-phenyl-benzthiazol-7-yl)-1,4,5,7-tetrahydrofuro[3,4-b]pyridin-3-carbonsäuremethylester

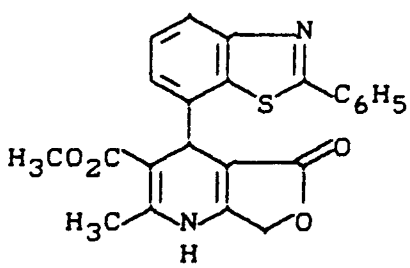

10 mmol 2-Phenyl-benzthiazol-7-carboxaldehyd, 10 mmol 4-Acetoxyacetessigester und 10 mmol 3-Aminocrotonsäuremethylester werden in 20 ml Methanol über Nacht gekocht, anschließend mit 6 ml verdünnter Salzsäure versetzt und 30 Minuten gekocht. Es wird eingeengt und an Kieselgel gesäult (Cyclohexan/Essigester = 1:1).
Ausbeute: 30%
Fp.: 275° C
Die in den Tabellen 7 und 8 aufgeführten Beispiele wurden in Analogie zur Vorschrift des Beispiels 27 hergestellt:

Tabelle 7

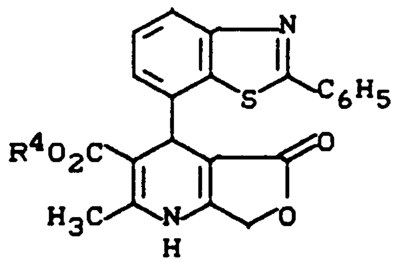

| Beispiel-Nr. | $R^4$ | F (°C) |
|---|---|---|
| 28 | $-C_2H_5$ | 165 |
| 29 | $-CH(CH_3)_2$ | 195 |

Tabelle 8

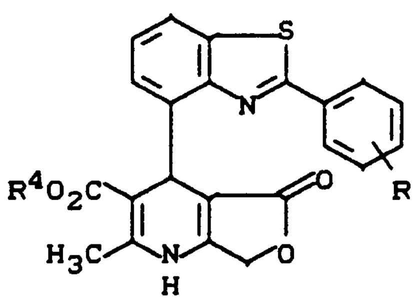

| Beispiel-Nr. | $R^4$ | R | F (°C) |
|---|---|---|---|
| 30 | $-CH_3$ | H | 285 |
| 31 | $-C_2H_5$ | H | 249 |
| 32 | $-CH(CH_3)_2$ | H | 210 |

Fortsetzung Tabelle 8

| Beispiel-Nr. | $R^4$ | R | F (°C) |
|---|---|---|---|
| 33 | $-C_2H_5$ | 2-Cl | 168 |
| 34 | $-C_2H_5$ | 3-F | 267 |
| 35 | $-C_2H_5$ | 4-F | 199 |
| 36 | $-C_2H_5$ | 2-F | 196 |
| 37 | $-C_2H_5$ | 3-Cl | 234 |
| 38 | $-C_2H_5$ | 4-Cl | 198 |

Die in Tabelle 9 aufgeführten Beispiele wurden in Analogie zur Vorschrift des Beispiels 1 hergestellt.

Tabelle 9:

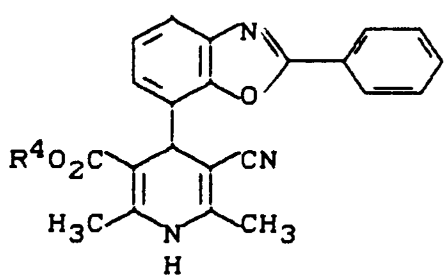

| Beispiel Nr. | $R^4$ | F°C |
|---|---|---|
| 39 | $-CH(CH_3)_2$ | 191 |
| 40 | $-CH_3$ | 199 |

Die in Tabelle 10 aufgeführten Beispiele wurden in Analogie zur Vorschrift des Beispiels 16 hergestellt.

Tabelle 10:

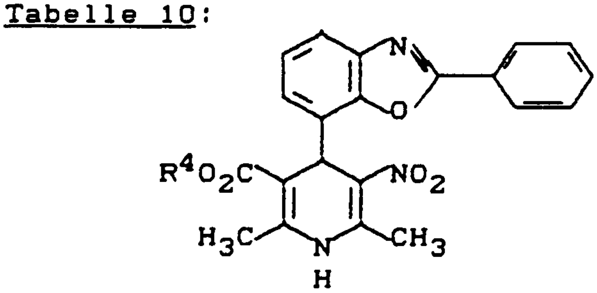

| Beispiel Nr. | $R^4$ | F° C |
|---|---|---|
| 41 | $-C_2H_5$ | 246 |
| 42 | $-CH_3$ | 245 |

Das in Tabelle 11 aufgeführte Beispiel wurde in Analogie zur Vorschrift des Beispiels 17 hergestellt.

Tabelle 10:

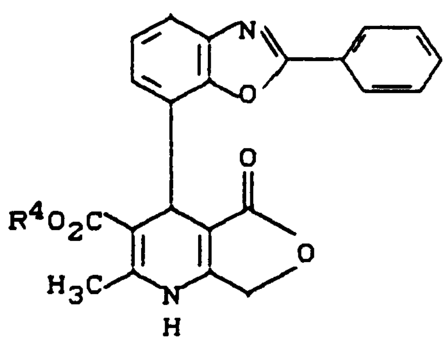

| Beispiel Nr. | $R^4$ | F° C |
|---|---|---|
| 43 | $-C_2H_5$ | 160 |

**Patentansprüche**

**Patentansprüche für folgende Vertragsstaaten : AT, BE, CH, DE, DK, FR, GB, GR, IT, LI, LU, NL, SE**

1. Heterocyclisch substituierte Dihydropyridine der allgemeinen Formel (I),

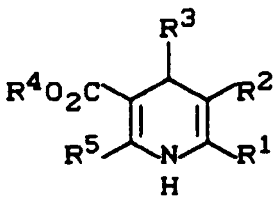

(I)

in welcher

$R^1$ und $R^5$ gleich oder verschieden sind und für geradkettiges oder verzweigtes Alkyl mit bis zu 8 Kohlenstoffatomen stehen,

$R^2$ für Nitro oder Cyano steht,

oder

$R^1$ und $R^2$ gemeinsam einen Lactonring der Formel

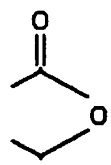

bilden,

$R^3$ für einen Rest der Formel

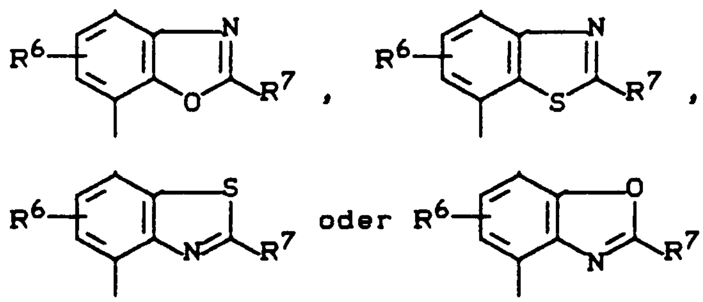

steht,

worin

$R^6$

- Wasserstoff, Halogen oder geradkettiges oder verzweigtes Alkyl oder Alkoxy mit jeweils bis zu 8 Kohlenstoffatomen bedeutet,

$R^7$

- Aryl mit 6 bis 10 Kohlenstoffatomen bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder
- Thienyl oder Pyridyl bedeutet,

$R^4$ für Wasserstoff steht, oder

für geradkettiges oder verzweigtes Alkyl, Alkenyl, Alkadienyl oder Alkinyl mit jeweils

bis zu 10 Kohlenstoffatomen steht, die gegebenenfalls bis zu 2-fach gleich oder verschieden durch Halogen, Hydroxy, Carboxy, Cyano, Nitro, Phenoxy oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können

und deren physiologisch unbedenklichen Salze.

**2.** Verbindungen der allgemeinen Formel (I) gemäß Anspruch 1, in welcher

$R^1$ und $R^5$ gleich oder verschieden sind und für geradkettiges oder verzweigtes Alkyl mit bis zu 6 Kohlenstoffatomen stehen,

$R^2$ für Nitro oder Cyano steht,

oder

$R^1$ und $R^2$ gemeinsam einen Lactonring der Formel

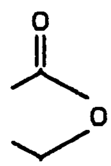

bilden,

$R^3$ für einen Rest der Formel

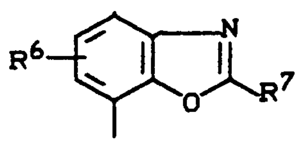
,
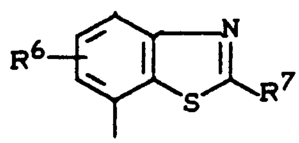
,

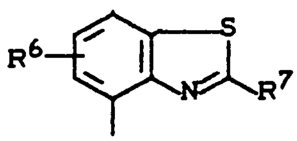
oder
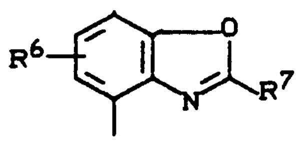

steht,

worin

$R^6$

- Wasserstoff, Fluor, Chlor oder geradkettiges oder verzweigtes Alkyl oder Alkoxy mit jeweils bis zu 2 Kohlenstoffatomen bedeutet,

$R^7$

- Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist, oder
- Pyridyl oder Thienyl bedeutet,

$R^4$

- für Wasserstoff oder für geradkettiges oder verzweigtes Alkyl oder Alkenyl mit jeweils bis zu 8 Kohlenstoffatomen steht, die gegebenenfalls durch Fluor, Chlor, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 6 Kohlenstoffatomen oder durch Phenoxy oder Phenyl substituiert sind

und deren physiologisch unbedenklichen Salze.

**3.** Verbindungen der allgemeinen Formel (I) gemäß Anspruch 1
in welcher

$R^1$ und $R^5$ gleich oder verschieden sind und
- für geradkettiges oder verzweigtes Alkyl mit bis zu 4 Kohlenstoffatomen stehen,

$R^2$ für Nitro oder Cyano steht,

oder

$R^1$ und $R^2$ gemeinsam einen Lactonring der Formel

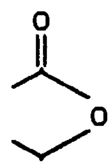

bilden,

$R^3$ für einen Rest der Formel

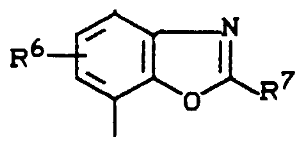
,
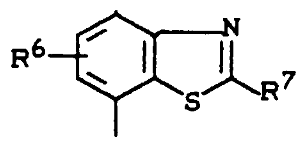
,
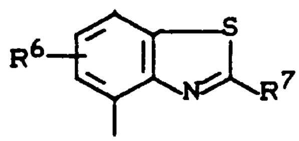
oder
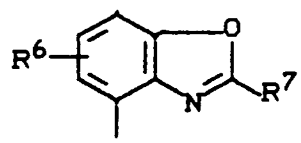

steht,
worin

$R^6$
- Wasserstoff, Chlor oder Methyl bedeutet,

$R^7$
- Phenyl bedeutet, das gegebenenfalls durch Fluor, Chlor, Nitro, Trifluormethyl oder
- geradkettiges oder verzweigtes Alkyl, Acyl oder Alkoxy mit jeweils bis zu 4 Kohlenstoffatomen substituiert ist, oder
- Pyridyl bedeutet

$R^4$
- für Wasserstoff steht, oder
- für geradkettiges oder verzweigtes Alkyl mit bis zu 6 Kohlenstoffatomen steht, das gegebenenfalls durch Hydroxy, Carboxy, Cyano oder durch geradkettiges oder verzweigtes Alkoxycarbonyl, Alkoxy oder Acyloxy mit jeweils bis zu 4 Kohlenstoffatomen substituiert ist

und deren physiologisch unbedenklichen Salze.

**4.** Verfahren zur Herstellung von Verbindungen der allgemeinen Formel (I)

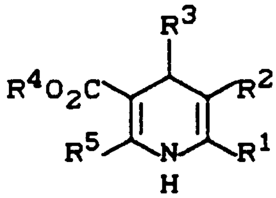

(I)

in welcher $R^1$, $R^2$, $R^3$, $R^4$, $R^5$, $R^6$, $R^7$, die in Anspruch 1 angegebene Bedeutung haben.
und deren physiologisch unbedenklichen Salze,
dadurch gekennzeichnet, daß man
daß man im Fall, daß $R^1$ und $R^2$ die oben angegebene Bedeutung haben, aber nicht gemeinsam einen Lactonring bilden,

[A] Verbindungen der allgemeinen Formel (II)

$R^3$-CHO (II)

in welcher
$R^3$ die oben angegebene Bedeutung hat,
zunächst mit Acetessigestern der allgemeinen Formel (III)

$R^5$-CO-$CH_2$-$CO_2$-$R^4$ (III)

in welcher
$R^4$ und $R^5$ die oben angegebene Bedeutung haben,
gegebenenfalls unter Isolierung der entsprechenden Ylidenverbindungen der allgemeinen Formel (IV)

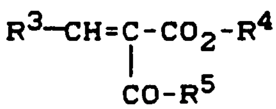

(IV)

in welcher
$R^3$, $R^4$ und $R^5$ die oben angegebene Bedeutung haben,
umsetzt und anschließend
entweder mit Verbindungen der Formel (V)

$R^1$-CO-$CH_2$-$R^2$ (V)

in welcher
$R^1$ und $R^2$ die oben angegebene Bedeutung haben,
in inerten Lösemitteln, in Anwesenheit von Ammoniak oder Ammoniumsalzen,
oder direkt mit Enaminoderivaten der allgemeinen Formel (VI)

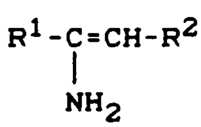

(VI)

in welcher
$R^1$ und $R^2$ die oben angegebene Bedeutung haben,
umsetzt,

oder

[B] die Aldehyde der allgemeinen Formel (II) zunächst mit den Verbindungen der allgemeinen Formel (V), gegebenenfalls unter Isolierung der Ylidenverbindungen der allgemeinen Formel (VII)

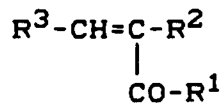 (VII)

in welcher

$R^1$, $R^2$ und $R^3$ die oben angegebene Bedeutung haben,

umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII)

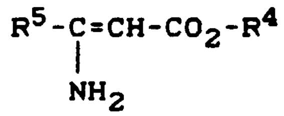 (VIII)

in welcher

$R^4$ und $R^5$ die oben angegebene Bedeutung haben,

umsetzt,

oder im Fall, daß $R^1$ und $R^2$ gemeinsam einen Lactonring bilden,

[C] zunächst nach denen unter [A] und [B] aufgeführten Methoden, Verbindungen der allgemeinen Formel (Ia)

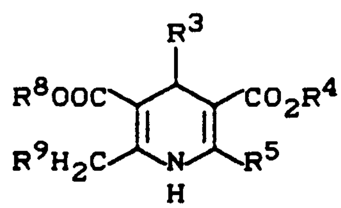 (Ia)

in welcher

$R^3$, $R^4$ und $R^5$ die oben angegebene Bedeutung haben,

$R^8$ für einen $C_1$-$C_6$-Alkyl-Rest steht

und

$R^9$ für eine Abgangsgruppe wie beispielsweise Chlor oder Acetoxy steht,

herstellt und nach bekannten Verfahren einen säure-oder basenkatalysierten Ringschluß anschließt,

und im Fall, daß $R^4$ nicht Wasserstoff bedeutet,

[D] Verbindungen der allgemeinen Formel (I), in welcher $R^1$, $R^2$, $R^3$, $R^4$ und $R^5$ die oben angegebene Bedeutung haben und $R^4$ für Wasserstoff steht, gegebenenfalls über ein reaktives Säurederivat, mit den entsprechenden Alkoholen umsetzt, wobei durch Einsatz der enantiomerenreinen Carbonsäure die entsprechenden Enantiomere der Ester erhalten werden,

**5.** Aldehyde der allgemeinen Formel (II)

$R^3$-CHO (II)

in welcher

$R^3$ für einen Rest der Formel

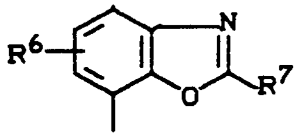 , 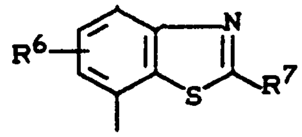 ,

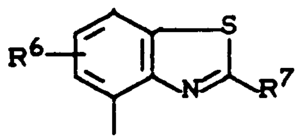 oder 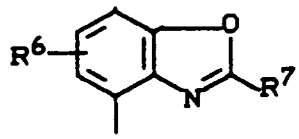

steht,
worin

$R^6$
- Wasserstoff, Fluor, Chlor oder geradkettiges oder verzweigtes Alkyl oder Alkoxy mit jeweils bis zu 2 Kohlenstoffatomen bedeutet,

$R^7$
- Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist, oder
- Pyridyl oder Thienyl bedeutet,

**6.** Verfahren zur Herstellung von Aldehyden der allgemeinen Formel (II) gemäß Anspruch 5, dadurch gekennzeichnet, daß man

a) entweder Verbindungen der allgemeinen Formel IX

$R^{3'}$-$CH_3$ (IX)

in welcher

$R^{3'}$ für die Reste der Formel

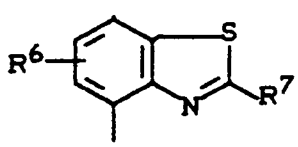 oder 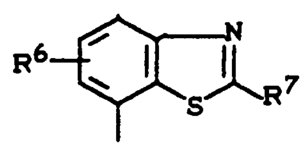

steht,

in welcher

$R^6$ und $R^7$ die oben angegebene Bedeutung haben,

zu Verbindungen der allgemeinen Formel (X)

$R^{3'}$-$CH_2$-Hal (X)

in welcher

$R^{3'}$ die oben angegebene Bedeutung hat

und

Hal für Halogen, vorzugsweise für Brom steht,

halogeniert, mit Acetationen umsetzt und anschließend zu Verbindungen der allgemeinen Formel (XI)

$R^{3'}$-$CH_2OH$ (XI)

in welcher

$R^{3'}$ die oben angegebene Bedeutung hat

verseift und in einem letzten Schritt nach üblicher Methode oxidiert,

b) oder indem man Verbindungen der allgemeinen Formel (XII)

$R^{3''}$-$CH_3$ (XII)

in welcher

$R^{3''}$ für einen Rest der Formel

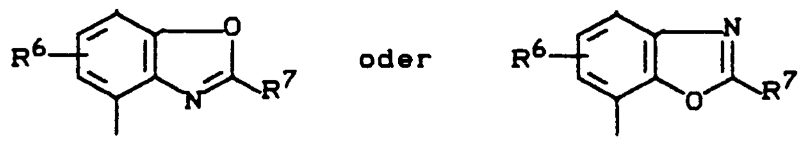

steht,

in welcher

$R^6$ und $R^7$ die oben angegebene Bedeutung haben,

zu Verbindungen der allgemeinen Formel (XIII)

$R^{3''}$-$CH(Br)_2$ (XIII)

in welcher

$R^{3''}$ die oben angegebene Bedeutung hat

halogeniert und anschließend nach üblicher Methode hydrolysiert.

**7.** Arzneimittel enthaltend mindestens eine Verbindung der allgemeinen Formel (I) gemäß Anspruch 1.

**8.** Verfahren zur Herstellung von Arzneimitteln, dadurch gekennzeichnet, daß man mindestens eine Verbindung der allgemeinen Formel (I) gemäß Anspruch 1, gegebenenfalls unter Verwendung von üblichen Hilfs- und Trägerstoffen in eine geeigneten Applikationsform überführt.

**9.** Verwendung von Verbindungen der allgemeinen Formel (I) gemäß Anspruch 1 bei der Herstellung von Arzneimitteln.

**Patentanspruch für folgenden Vertragsstaat : ES**

**1.** Verfahren zur Herstellung von Verbindungen der allgemeinen Formel (I)

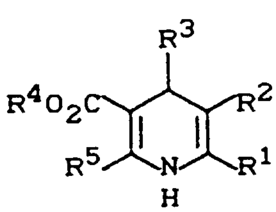

(I)

in welcher

$R^1$ und $R^5$ gleich oder verschieden sind und für geradkettiges oder verzweigtes Alkyl mit bis zu 8 Kohlenstoffatomen stehen,

$R^2$ für Nitro oder Cyano steht,

oder

$R^1$ und $R^2$ gemeinsam einen Lactonring der Formel

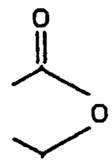

bilden,

$R^3$ für einen Rest der Formel

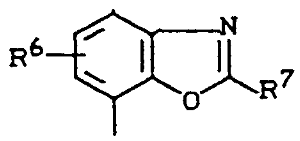 , 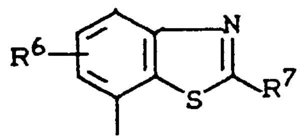 ,

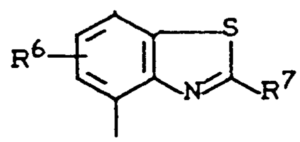 oder 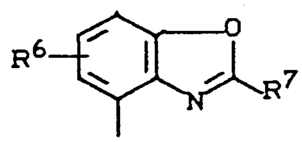

steht,

worin

$R^6$

- Wasserstoff, Halogen oder geradkettiges oder verzweigtes Alkyl oder Alkoxy mit jeweils bis zu 8 Kohlenstoffatomen bedeutet,

$R^7$

- Aryl mit 6 bis 10 Kohlenstoffatomen bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder
- Thienyl oder Pyridyl bedeutet,

$R^4$ für Wasserstoff steht, oder für geradkettiges oder verzweigtes Alkyl, Alkenyl, Alkadienyl oder Alkinyl mit jeweils bis zu 10 Kohlenstoffatomen steht, die gegebenenfalls bis zu 2-fach gleich oder verschieden durch Halogen, Hydroxy, Carboxy, Cyano, Nitro, Phenoxy oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können

und deren physiologisch unbedenklichen Salze,
dadurch gekennzeichnet,
daß man im Fall, daß $R^1$ und $R^2$ die oben angegebene Bedeutung haben, aber nicht gemeinsam einen Lactonring bilden,

[A] Verbindungen der allgemeinen Formel (II)

$R^3$-CHO (II)

in welcher

$R^3$ die oben angegebene Bedeutung hat,

zunächst mit Acetessigestern der allgemeinen Formel (III)

$R^5$-CO-$CH_2$-$CO_2$-$R^4$ (III)

in welcher

$R^4$ und $R^5$ die oben angegebene Bedeutung haben,

gegebenenfalls unter Isolierung der entsprechenden Ylidenverbindungen der allgemeinen Formel (IV)

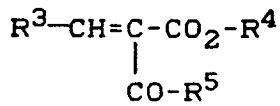

(IV)

in welcher

$R^3$, $R^4$ und $R^5$ die oben angegebene Bedeutung haben,

umsetzt und anschließend

entweder mit Verbindungen der Formel (V)

$R^1$-CO-$CH_2$-$R^2$ (V)

in welcher

$R^1$ und $R^2$ die oben angegebene Bedeutung haben,

in inerten Lösemitteln, in Anwesenheit von Ammoniak oder Ammoniumsalzen,

oder direkt mit Enaminoderivaten der allgemeinen Formel (VI)

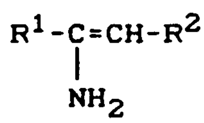

(VI)

in welcher

$R^1$ und $R^2$ die oben angegebene Bedeutung haben,

umsetzt,

oder

[B] die Aldehyde der allgemeinen Formel (II) zunächst mit den Verbindungen der allgemeinen Formel (V), gegebenenfalls unter Isolierung der Ylidenverbindungen der allgemeinen Formel (VII)

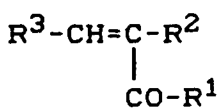

(VII)

in welcher

$R^1$, $R^2$ und $R^3$ die oben angegebene Bedeutung haben,

umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII)

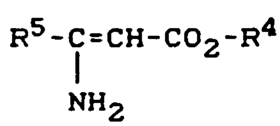

(VIII)

in welcher

$R^4$ und $R^5$ die oben angegebene Bedeutung haben,

umsetzt,

oder im Fall, daß $R^1$ und $R^2$ gemeinsam einen Lactonring bilden,

[C] zunächst nach denen unter [A] und [B] aufgeführten Methoden, Verbindungen der allgemeinen Formel (Ia)

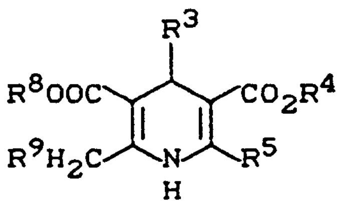

(Ia)

in welcher

$R^3$, $R^4$ und $R^5$ die oben angegebene-Bedeutung haben,

$R^8$ für einen $C_1$-$C_6$-Alkyl-Rest steht

und

$R^9$ für eine Abgangsgruppe wie beispielsweise Chlor oder Acetoxy steht,

herstellt und nach bekannten Verfahren einen säure-oder basenkatalysierten Ringschluß anschließt, und im Fall, daß $R^4$ nicht Wasserstoff bedeutet,

[D] Verbindungen der allgemeinen Formel (I), in welcher $R^1$, $R^2$, $R^3$, $R^4$ und $R^5$ die oben angegebene Bedeutung haben und $R^4$ für Wasserstoff steht, gegebenenfalls über ein reaktives Säurederivat, mit den entsprechenden Alkoholen umsetzt, wobei durch Einsatz der enantiomerenreinen Carbonsäure die entsprechenden Enantiomere der Ester erhalten werden.

## Claims

**Claims for the following Contracting States : AT, BE, CH, DE, DK, FR, GB, GR, IT, LI, LU, NL, SE**

**1.** Dihydropyridines substituted by heterocycles, of the general formula (I)

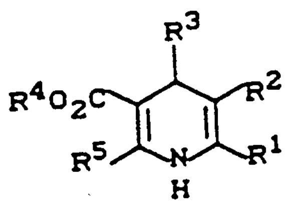

(I)

in which

$R^1$ and $R^5$ are identical or different and represent straight-chain or branched alkyl having up to 8 carbon atoms,

$R^2$ represents nitro or cyano,

or

$R^1$ and $R^2$ together form a lactone ring of the formula

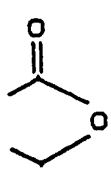

$R^3$ represents a radical of the formula

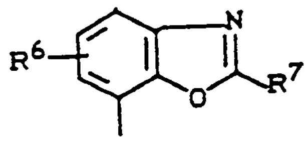
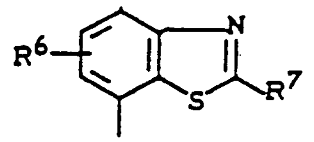 ,

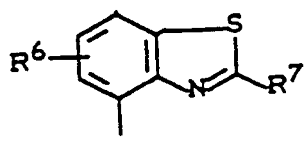 or 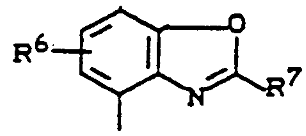 ,

in which

R⁶
- denotes hydrogen, halogen or straight-chain or branched alkyl or alkoxy in each case having up to 8 carbon atoms,

R⁷
- denotes aryl having 6 to 10 carbon atoms, which is optionally monosubstituted or disubstituted by identical or different substituents from the series comprising halogen, nitro, cyano, trifluoromethyl, trifluoromethoxy, trifluoromethylthio, straight-chain or branched alkyl, alkoxy or alkoxycarbonyl in each case having up to 8 carbon atoms, and carboxyl, or
- denotes thienyl or pyridyl,

$R^4$ represents hydrogen, or
represents straight-chain or branched alkyl, alkenyl, alkadienyl or alkinyl in each case having up to 10 carbon atoms which are optionally monosubstituted or disubstituted by identical or different substituents from the series comprising halogen, hydroxyl, carboxyl, cyano, nitro, phenoxy, straight-chain or branched alkylthio, alkoxy, alkoxycarbonyl, acyl or acyloxy in each case having up to 8 carbon atoms, and phenoxy or phenyl, it being possible for the latter in turn to be monosubstituted or disubstituted by identical or different substituents from the series comprising halogen and straight-chain or branched alkyl or alkoxy in each case having up to 6 carbon atoms

and their physiologically acceptable salts.

**2.** Compounds of the general formula (I) according to Claim 1,
in which

$R^1$ and $R^5$ are identical or different and represent straight-chain or branched alkyl having up to 6 carbon atoms,

$R^2$ represents nitro or cyano,

or

$R^1$ and $R^2$ together form a lactone ring of the formula

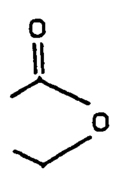

$R^3$ represents a radical of the formula

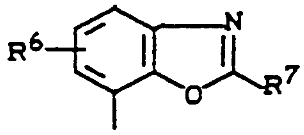 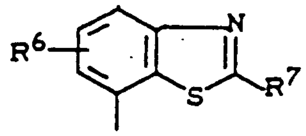 ,

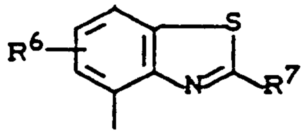 or 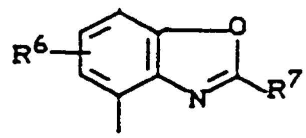

in which

$R^6$
- denotes hydrogen, fluorine, chlorine or straight-chain or branched alkyl or alkoxy in each case having up to 2 carbon atoms,

$R^7$
- denotes phenyl which is optionally substituted by fluorine, chlorine, nitro, cyano, trifluoromethyl or by straight-chain or branched alkyl or alkoxy in each case having up to 6 carbon atoms, or
- denotes pyridyl or thienyl,

$R^4$
- represents hydrogen or
represents straight-chain or branched alkyl or alkenyl in each case having up to 8 carbon atoms which are optionally substituted by fluorine, chlorine, hydroxyl, carboxyl, cyano, nitro or by straight-chain or branched alkylthio, alkoxy, alkoxycarbonyl, acyl or acyloxy in each case having up to 6 carbon atoms or by phenoxy or phenyl

and their physiologically acceptable salts.

**3.** Compounds of the general formula (I) according to Claim 1
in which

$R^1$ and $R^5$ are identical or different and
- represent straight-chain or branched alkyl having up to 4 carbon atoms,

$R^2$ represents nitro or cyano,

or

$R^1$ and $R^2$ together form a lactone ring of the formula

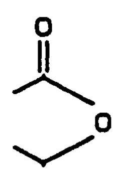

$R^3$ represents a radical of the formula

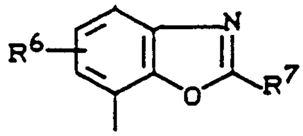

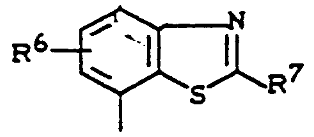,

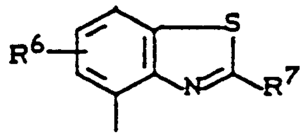

or

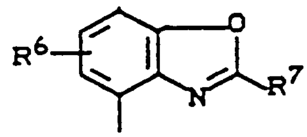,

in which

$R^6$ - denotes hydrogen, chlorine or methyl,

$R^7$ - denotes phenyl which is optionally substituted by fluorine, chlorine, nitro, trifluoromethyl or straight-chain or branched alkyl, acyl or alkoxy in each case having up to 4 carbon atoms, or
- denotes pyridyl

$R^4$ - represents hydrogen, or
- represents straight-chain or branched alkyl having up to 6 carbon atoms, which is optionally substituted by hydroxyl, carboxyl, cyano or by straight-chain or branched alkoxycarbonyl, alkoxy or acyloxy in each case having up to 4 carbon atoms

and their physiologically acceptable salts.

**4.** Process for the preparation of compounds of the general formula (I)

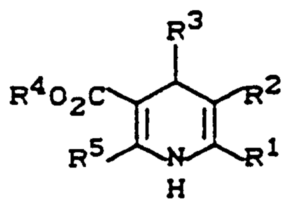

(I)

in which $R^1$, $R^2$, $R^3$, $R^4$, $R^5$, $R^6$ and $R^7$ have the meaning given in Claim 1, and their physiologically acceptable salts, characterized in that in the case in which $R^1$ and $R^2$ have the above-mentioned meaning, but do not together form a lactone ring,

[A] compounds of the general formula (II)

$R^3$-CHO (II)

in which

$R^3$ has the abovementioned meaning,

are first reacted with acetoacetic esters of the general formula (III)

$R^5$-CO-$CH_2$-$CO_2$-$R^4$ (III)

in which

$R^4$ and $R^5$ have the abovementioned meaning,

if desired with the isolation of the corresponding ylidene compounds of the general formula (IV)

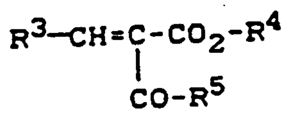

(IV)

in which

$R^3$, $R^4$ and $R^5$ have the abovementioned meaning, and then

are reacted either with compounds of the formula (V)

$R^1$-CO-$CH_2$-$R^2$ (V)

in which

$R^1$ and $R^2$ have the abovementioned meaning,

in inert solvents, in the presence of ammonia or ammonium salts,

or directly with enamino derivatives of the general formula (VI)

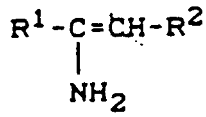

(VI)

in which

$R^1$ and $R^2$ have the abovementioned meaning,

or

[B] the aldehydes of the general formula (II) are first reacted with the compounds of the general formula (V), if desired with the isolation of the ylidene compounds of the general formula (VII)

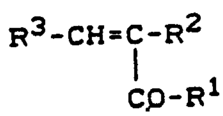

(VII)

in which

$R^1$, $R^2$ and $R^3$ have the abovementioned meaning,

and in a next step reacted with the above-mentioned compounds of the general formula (III) in inert solvents, in the presence of ammonia or ammonium salts or directly with enaminocarboxylic acid derivatives of the general formula (VIII)

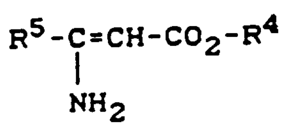

(VIII)

in which

$R^4$ and $R^5$ have the abovementioned meaning,

or in the case in which $R^1$ and $R^2$ together form a lactone ring,

[C] first, according to those methods given under [A] and [B], compounds of the general formula (Ia)

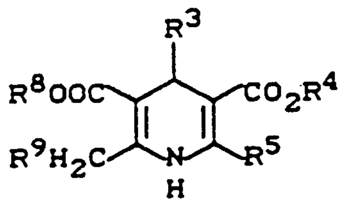

(Ia)

in which

$R^3$, $R^4$ and $R^5$ have the abovementioned meaning,

$R^8$ represents a $C_1$-$C_6$-alkyl radical

and

$R^9$ represents a leaving group such as, for example, chlorine or acetoxy,

are prepared and, according to known methods, an acid- or base-catalysed ring closure is added, and in the case in which $R^4$ does not denote hydrogen,

[D] compounds of the general formula (I), in which $R^1$, $R^2$, $R^3$, $R^4$ and $R^5$ have the abovementioned meaning and $R^4$ represents hydrogen, are reacted with the corresponding alcohols, if appropriate via a reactive acid derivative, in which case by use of the enantiomerically pure carboxylic acid the corresponding enantiomers of the esters are obtained.

**5.** Aldehydes of the general formula (II)

$R^3$-CHO (II)

in which

$R^3$ represents a radical of the formula

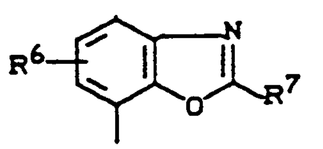

,

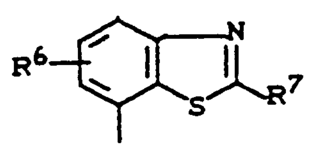

,

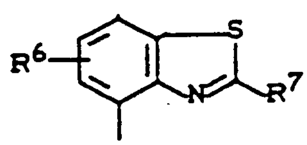

or

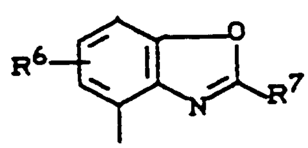

in which

$R^6$

- denotes hydrogen, fluorine, chlorine or straight-chain or branched alkyl or alkoxy in each case having up to 2 carbon atoms,

$R^7$

- denotes phenyl which is optionally substituted by fluorine, chlorine, nitro, cyano, trifluoromethyl or by straight-chain or branched alkyl or alkoxy in each case having up to 6 carbon atoms, or
- denotes pyridyl or thienyl.

**6.** Process for the preparation of aldehydes of the general formula (II) according to Claim 5, characterized in that

a) either compounds of the general formula (IX)

$R^{3'}$-$CH_3$ (IX)

in which

$R^{3'}$ represents the radicals of the formula

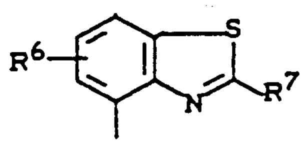 or 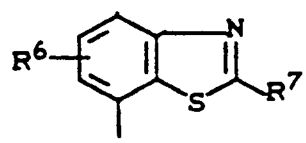 ,

in which

$R^6$ and $R^7$ have the abovementioned meaning,

are halogenated to give compounds of the general formula (X)

$R^{3'}$ $-CH_2-Hal$ (X)

in which

$R^{3'}$ has the abovementioned meaning

and

Hal represents halogen, preferably bromine,

reacted with acetate ions and then hydrolysed to give compounds of the general formula (XI)

$R^{3'}$ $-CH_2OH$ (XI)

in which

$R^{3'}$ has the abovementioned meaning

and in a last step oxidized by a customary method,

b) or compounds of the general formula (XII)

$R^{3''}$ $-CH_3$ (XII)

in which

$R^{3''}$ represents a radical of the formula

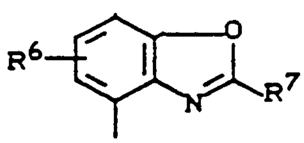 or 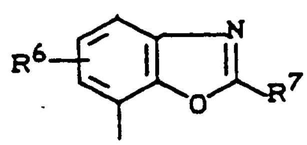 ,

in which

$R^6$ and $R^7$ have the abovementioned meaning,

are halogenated to give compounds of the general formula (XIII)

$R^{3''}$ $-CH(Br)_2$ (XIII)

in which

$R^{3''}$ has the abovementioned meaning

and then hydrolysed by a customary method.

**7.** Medicaments containing at least one compound of the general formula (I) according to Claim 1.

**8.** Process for the production of medicaments, characterized in that at least one compound of the general formula (I) according to Claim 1 is converted into a suitable administration form, if appropriate using customary auxiliaries and excipients.

**9.** Use of compounds of the general formula (I) according to Claim 1 in the production of medicaments.

**Claim for the following Contracting State : ES**

**1.** Process for the preparation of compounds of the general formula (I)

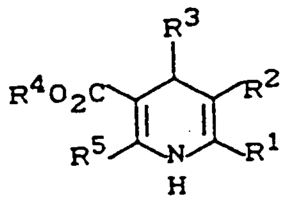

(I)

in which

$R^1$ and $R^5$ are identical or different and represent straight-chain or branched alkyl having up to 8 carbon atoms,

$R^2$ represents nitro or cyano,

or

$R^1$ and $R^2$ together form a lactone ring of the formula

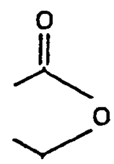

$R^3$ represents a radical of the formula

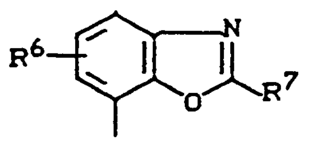 , 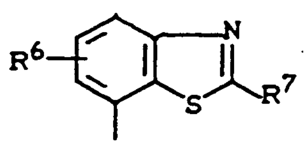 ,

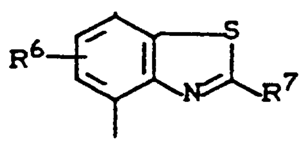 or 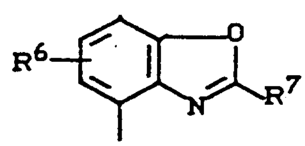 ,

in which

$R^6$

- denotes hydrogen, halogen or straight-chain or branched alkyl or alkoxy in each case having up to 8 carbon atoms,

$R^7$

- denotes aryl having 6 to 10 carbon atoms, which is optionally monosubstituted or disubstituted by identical or different substituents from the series comprising halogen, nitro, cyano, trifluoromethyl, trifluoromethoxy, trifluoromethylthio, straight-chain or branched alkyl, alkoxy or alkoxycarbonyl in each case having up to 8 carbon atoms, and carboxyl, or
- denotes thienyl or pyridyl,

$R^4$ represents hydrogen, or

represents straight-chain or branched alkyl, alkenyl, alkadienyl or alkinyl in each case having up to 10 carbon atoms which are optionally monosubstituted or disubstituted by identical or different substituents from the series comprising halogen, hydroxyl, carboxyl, cyano, nitro, phenoxy and straight-chain or branched alkylthio, alkoxy, alkoxycarbonyl, acyl or acyloxy in each case having up to 8 carbon atoms, and phenoxy or phenyl, it being possible for the latter in turn to be monosubstituted or

disubstituted by identical or different substituents from the series comprising halogen and straight-chain or branched alkyl or alkoxy in each case having up to 6 carbon atoms

and their physiologically acceptable salts,
characterized in that
in the case in which $R^1$ and $R^2$ have the abovementioned meaning, but do not together form a lactone ring,

[A] compounds of the general formula (II)

$R^3$-CHO (II)

in which

$R^3$ has the abovementioned meaning,

are first reacted with acetoacetic esters of the general formula (III)

$R^5-CO-CH_2-CO_2-R^4$ (III)

in which

$R^4$ and $R^5$ have the abovementioned meaning,

if desired with the isolation of the corresponding ylidene compounds of the general formula (IV)

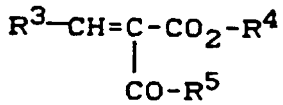

(IV)

in which

$R^3$, $R^4$ and $R^5$ have the abovementioned meaning,

and then

are reacted either with compounds of the formula (V)

$R^1-CO-CH_2-R^2$ (V)

in which

$R^1$ and $R^2$ have the abovementioned meaning,

in inert solvents, in the presence of ammonia or ammonium salts,

or directly with enamino derivatives of the general formula (VI)

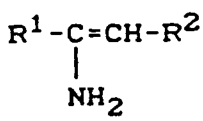

(VI)

in which

$R^1$ and $R^2$ have the abovementioned meaning,

or

[B] the aldehydes of the general formula (II) are first reacted with the compounds of the general formula (V), if desired with the isolation of the ylidene compounds of the general formula (VII)

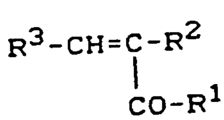

(VII)

in which

$R^1$, $R^2$ and $R^3$ have the abovementioned meaning, and in a next step reacted with the abovementioned compounds of the general formula (III) in inert solvents, in the presence of ammonia or ammonium salts or directly with enaminocarboxylic acid derivatives of the general formula (VIII)

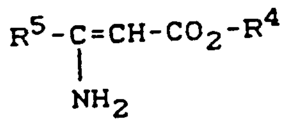

(VIII)

in which

$R^4$ and $R^5$ have the abovementioned meaning,

or in the case in which $R^1$ and $R^2$ together form a lactone ring,

[C] first, according to those methods given under [A] and [B], compounds of the general formula (Ia)

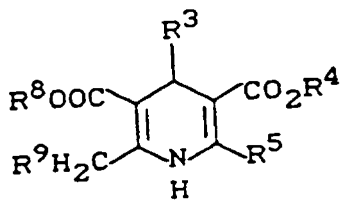

(Ia)

in which

$R^3$, $R^4$ and $R^5$ have the abovementioned meaning,

$R^8$ represents a $C_1$-$C_6$-alkyl radical

and

$R^9$ represents a leaving group such as, for example, chlorine or acetoxy,

are prepared and, according to known methods, an acid- or base-catalysed ring closure is added,

and in the case in which $R^4$ does not denote hydrogen,

[D] compounds of the general formula (I), in which $R^1$, $R^2$, $R^3$, $R^4$ and $R^5$ have the abovementioned meaning and $R^4$ represents hydrogen, are reacted with the corresponding alcohols, if appropriate via a reactive acid derivative, in which case by use of the enantiomerically pure carboxylic acid the corresponding enantiomers of the esters are obtained.

**Revendications**

**Revendications pour les Etats contractants suivants : AT, BE, CH, DE, DK, FR, GB, GR, IT, LI, LU, NL, SE**

**1.** Dihydropyridines substituées par des hétérocycles, de formule générale (I),

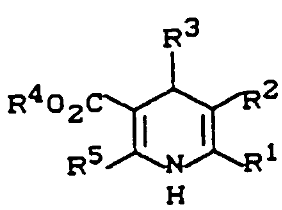

(I)

dans laquelle

$R^1$ et $R^5$ sont identiques ou différents et représentent un groupe alkyle à chaîne droite ou ramifié ayant jusqu'à 8 atomes de carbone,

$R^2$ est un groupe nitro ou cyano,

ou bien

$R^1$ et $R^2$ forment ensemble un noyau de lactone de formule

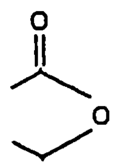

$R^3$ est un reste de formule

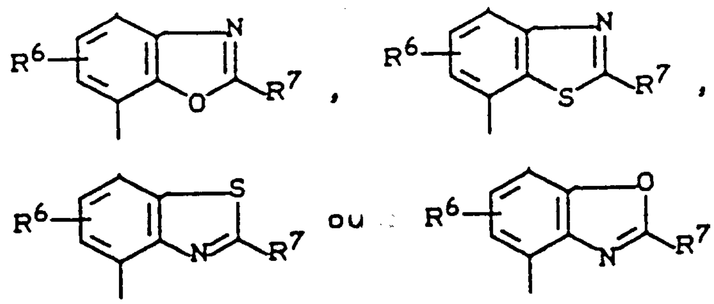

dans laquelle

$R^6$
- représente de l'hydrogène, un halogène ou un groupe alkyle ou alkoxy linéaire ou ramifié ayant chacun jusqu'à 8 atomes de carbone,

$R^7$
- est un groupe aryle ayant 6 à 10 atomes de carbone, qui porte le cas échéant jusqu'à 2 substituants, identiques ou différents, halogéno, nitro, cyano, trifluorométhyle, trifluorométhoxy, trifluorométhylthio, alkyle, alkoxy ou alkoxycarbonyle linéaire ou ramifié ayant chacun jusqu'à 8 atomes de carbone, ou carboxy, ou bien
- un groupe thiényle ou pyridyle,

$R^4$ représente de l'hydrogène ou un groupe alkyle, alcényle, alcadiényle ou alcynyle linéaire ou ramifié ayant chacun jusqu'à 10 atomes de carbone, qui portent le cas échéant jusqu'à 2 substituants identiques ou différents, halogéno, hydroxy, carboxy, cyano, nitro, phénoxy, ou alkylthio, alkoxy, alkoxycarbonyle, acyle ou acyloxy linéaire ou ramifié ayant chacun jusqu'à 8 atomes de carbone, ou phénoxy ou phényle, ces derniers pouvant être substitués quant à eux jusqu'à 2 fois identiques ou différentes par un halogène ou par un radical alkyle ou alkoxy linéaire ou ramifié ayant chacun jusqu'à 6 atomes de carbone,

et leurs sels acceptables du point de vue physiologique.

**2.** Composés de formule générale (I) suivant la revendication 1, formule dans laquelle

$R^1$ et $R^5$ sont identiques ou différents et représentent un groupe alkyle linéaire ou ramifié ayant jusqu'à 6 atomes de carbone,

$R^2$ est un groupe nitro ou cyano,

ou bien

$R^1$ et $R^2$ forment ensemble un noyau de lactone de formule

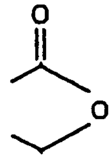

R$^3$ est un reste de formule

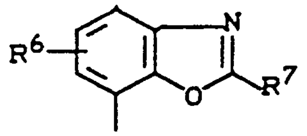 , 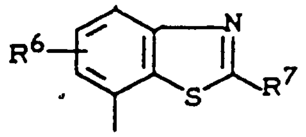 ,

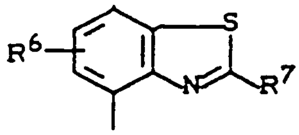 ou 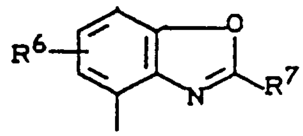

dans laquelle

R$^6$

- est de l'hydrogène, du fluor, du chlore ou un groupe alkyle ou alkoxy linéaire ou ramifié ayant chacun jusqu'à 2 atomes de carbone,

R$^7$

- est un groupe phényle qui est substitué le cas échéant par du fluor, du chlore, un radical nitro, cyano, trilfluorométhyle ou par un radical alkyle ou alkoxy linéaire ou ramifié ayant chacun jusqu'à 6 atomes de carbone, ou
- un groupe pyridyle ou thiényle,

R$^4$ est de l'hydrogène ou un groupe alkyle ou alcényle linéaire ou ramifié ayant chacun jusqu'à 8 atomes de carbone, qui sont substitués le cas échéant par un radical fluoro, chloro, hydroxy, carboxy, cyano, nitro ou par un radical alkylthio, alkoxy, alkoxycarbonyle, acyle ou acyloxy linéaire ou ramifié ayant chacun jusqu'à 6 atomes de carbone ou par un radical phénoxy ou phényle

et leurs sels acceptables du point de vue physiologique.

**3.** Composés de formule générale (I) suivant la revendication 1, dans laquelle

R$^1$ et R$^5$ sont identiques ou différents et représentent un groupe alkyle linéaire ou ramifié ayant jusqu'à 4 atomes de carbone,

R$^2$ est un groupe nitro ou cyano,

ou bien

R$^1$ et R$^2$ forment ensemble un noyau de lactone de formule

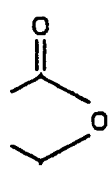

R$^3$ est un reste de formule

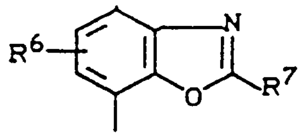 , 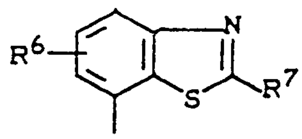 ,

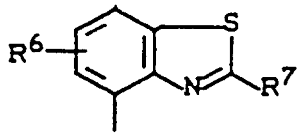 ou 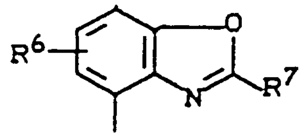

dans laquelle

$R^6$

- est de l'hydrogène, du chlore ou un groupe méthyle,

$R^7$

- est un groupe phényle qui est substitué le cas échéant par du fluor, du chlore, un radical nitro, trilfluorométhyle ou
- un radical alkyle, acyle ou alkoxy linéaire ou ramifié ayant chacun jusqu'à 4 atomes de carbone, ou représente
- un groupe pyridyle

$R^4$ est de l'hydrogène ou un groupe alkyle linéaire ou ramifié ayant jusqu'à 6 atomes de carbone, qui est substitué le cas échéant par un radical hydroxy, carboxy, cyano ou par un radical alkoxycarbonyle, alkoxy ou acyloxy linéaire ou ramifié ayant chacun jusqu'à 4 atomes de carbone

et leurs sels acceptables du point de vue physiologique.

**4.** Procédé de production de composés de formule générale (I)

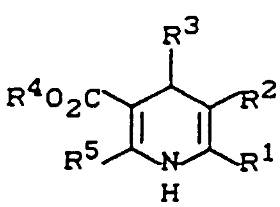 (I)

dans laquelle

$R^1$, $R^2$, $R^3$, $R^4$, $R^5$, $R^6$, $R^7$ ont la définition indiquée dans la revendication 1

et de leurs sels acceptables du point de vue physiologique, caractérisé en ce que

au cas où $R^1$ et $R^2$ ont la définition indiquée ci-dessus mais ne forment pas ensemble un noyau de lactone,

[A] on fait réagir des composés de formule générale (II)

$R^3$-CHO (II)

dans laquelle

$R^3$ à la définition indiquée ci-dessus,

tout d'abord avec des esters acétylacétiques de formule générale (III)

$R^5-CO-CH_2-CO_2-R^4$ (III)

dans laquelle

$R^4$ et $R^5$ ont la défintion indiquée ci-dessus,

le cas échéant avec isolement des composés ylidéniques correspondants de formule générale (IV)

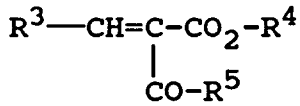
(IV)

dans laquelle
$R^3$, $R^4$ et $R^5$ ont la définition indiquée ci-dessus,
puis
on fait réagir les composés obtenus avec des composés de formule (V)

$R^1$-CO-$CH_2$-$R^2$ (V)

dans laquelle
$R^1$ et $R^2$ ont la définition indiquée ci-dessus,
dans des solvants inertes en présence d'ammoniac ou de sels d'ammonium,
ou directement avec des dérivés de type énamino de formule générale (VI)

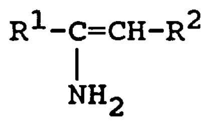
(VI)

dans laquelle
$R^1$ et $R^2$ ont la définition indiquée ci-dessus,
ou bien
[B] on fait réagir les aldéhydes de formule générale (II) tout d'abord avec les composés de formule générale (V), le cas échéant avec isolement du composé ylidénique de formule générale (VII)

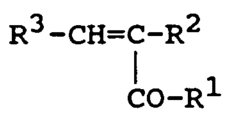
(VII)

dans laquelle
$R^1$, $R^2$ et $R^3$ ont la définition indiquée ci-dessus
et dans une étape suivante, on les fait réagir avec des composés de formule générale (III) indiqués ci-dessus dans des solvants inertes, en présence d'ammoniac ou de sels d'ammonium ou directement avec des dérivés d'acides énamino-carboxyliques de formule générale (VIII)

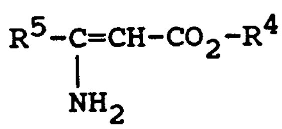
(VIII)

dans laquelle
$R^4$ et $R^5$ ont la définition indiquée ci-dessus,
ou bien au cas où $R^1$ et $R^2$ forment ensemble un noyau de lactone
[C] on prépare tout d'abord par les procédés indiqués en [A] et [B] des composés de formule générale (Ia)

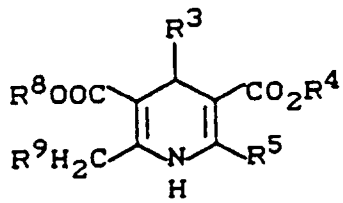

(Ia)

dans laquelle

$R^3$, $R^4$ et $R^5$ ont la définition indiquée ci-dessus,

$R^8$ représente un reste alkyle en $C_1$ à $C_6$

et

$R^9$ est un groupe partant tel que, par exemple, du chlore ou un groupe acétoxy,

et on effectue ensuite par des procédés connus une cyclisation catalysée par un acide ou par une base,

et au cas où $R^4$ ne représente pas de l'hydrogène,

[D] on fait réagir des composés de formule générale (I), dans laquelle $R^1$, $R^2$, $R^3$, $R^4$ et $R^5$ ont la définition indiquée ci-dessus et $R^4$ représente de l'hydrogène, le cas échéant en passant par un dérivé acide réactif, avec les alcools correspondants, et on obtient alors en utilisant l'acide carboxylique de pureté énantiomérique les énantiomères correspondants des esters.

**5.** Aldéhydes de formule générale (II)

$R^3$-CHO (II)

dans laquelle

$R^3$ représente un reste de formule

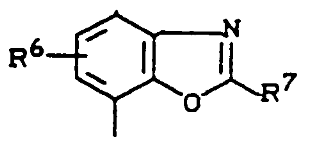 , 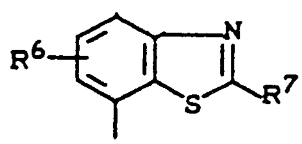 ,

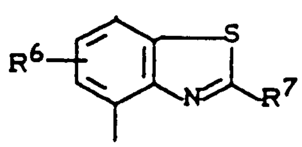 ou 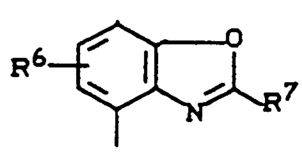

où

$R^6$

- représente de l'hydrogène, du fluor, du chlore ou un groupe alkyle ou alkoxy linéaire ou ramifié ayant chacun jusqu'à 2 atomes de carbone,

$R^7$

- est un groupe phényle qui est substitué le cas échéant par du fluor, du chlore, un radical nitro, cyano, trifluorométhyle ou par un radical alkyle ou alkoxy linéaire ou ramifié ayant chacun jusqu'à 6 atomes de carbone, ou bien
- un groupe pyridyle ou thiényle.

**6.** Procédé de production d'aldéhydes de formule générale (II) suivant la revendication 5, caractérisé en ce que

a) on halogène des composés de formule générale (IX)

$R^{3'}$-$CH_3$ (IX)

dans laquelle

$R^{3'}$ représente les restes de formule

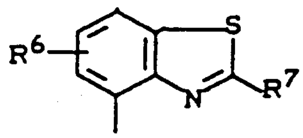

ou

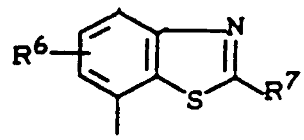

où

$R^6$ et $R^7$ ont la définition indiquée ci-dessus,

en composés de formule générale (X)

$R^{3'}$-$CH_2$-Hal (X)

dans laquelle

$R^{3'}$ a la définition indiquée ci-dessus

et

Hal représente un halogène, de préférence le brome,

on fait réagir les composés halogénés avec des ions acétate puis on les saponifie en composés de formule générale (XI)

$R^{3'}$-$CH_2OH$ (XI)

dans laquelle

$R^{3'}$ a la définition indiquée ci-dessus et on les oxyde dans une dernière étape par un procédé classique, ou bien

on halogène des composés de formule générale (XII)

$R^{3''}$-$CH_3$ (XII)

dans laquelle

$R^{3''}$ représente un reste de formule

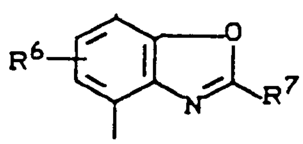

ou

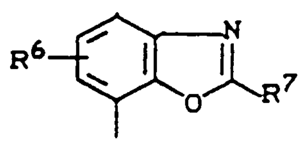

dans laquelle

$R^6$ et $R^7$ ont la définition indiquée ci-dessus,

en composés de formule générale (XIII)

$R^{3''}$-$CH(Br)_2$ (XIII)

dans laquelle

$R^{3''}$ a la définition indiquée ci-dessus, puis on hydrolyse les produits halogénés par un procédé classique.

**7.** Médicament contenant au moins un composé de formule générale (I) suivant la revendication 1.

**8.** Procédé de préparation de médicaments, caractérisé en ce qu'on transforme au moins un composé de formule générale (I) suivant la revendication 1, le cas échéant en utilisant des substances auxiliaires et des supports classiques, en une forme administrable appropriée.

9. Utilisation de composés de formule générale (I) suivant la revendication 1 pour la préparation de médicaments.

**Revendication pour l'Etat contractant suivant : ES**

1. Procédé de production de composés de formule générale (I),

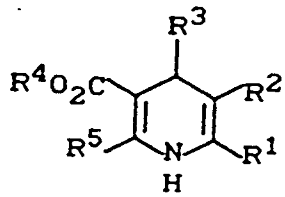

(I)

dans laquelle

$R^1$ et $R^5$ sont identiques ou différents et représentent un groupe alkyle à chaîne droite ou ramifié ayant jusqu'à 8 atomes de carbone,

$R^2$ est un groupe nitro ou cyano,

ou bien

$R^1$ et $R^2$ forment ensemble un noyau de lactone de formule

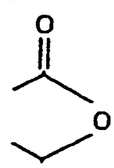

$R^3$ est un reste de formule

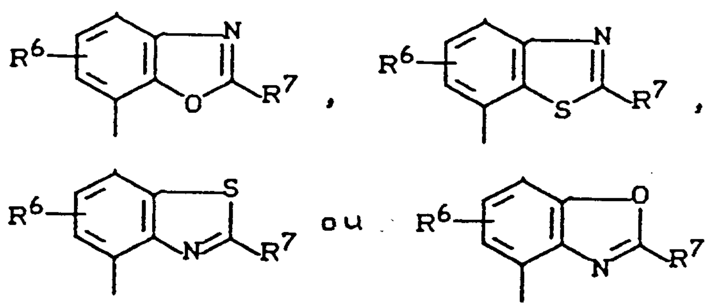

dans laquelle

$R^6$
- représente de l'hydrogène, un halogène ou un groupe alkyle ou alkoxy linéaire ou ramifié ayant chacun jusqu'à 8 atomes de carbone,

$R^7$
- est un groupe aryle ayant 6 à 10 atomes de carbone, qui porte le cas échéant jusqu'à 2 substituants, identiques ou différents, halogéno, nitro, cyano, trifluorométhyle, trifluorométhoxy, trifluorométhylthio, alkyle, alkoxy ou alkoxycarbonyle linéaire ou ramifié ayant chacun jusqu'à 8 atomes de carbone, ou carboxy, ou bien
- un groupe thiényle ou pyridyle,

$R^4$ représente de l'hydrogène ou un groupe alkyle, alcényle, alcadiényle ou alcynyle linéaire ou ramifié ayant chacun jusqu'à 10 atomes de carbone, qui portent le cas échéant jusqu'à 2 substituants identiques ou

différents, halogéno, hydroxy, carboxy, cyano, nitro, phénoxy, ou alkylthio, alkoxy, alkoxycarbonyle, acyle ou acyloxy linéaire ou ramifié ayant chacun jusqu'à 8 atomes de carbone, ou phénoxy ou phényle, ces derniers pouvant être substitués quant à eux jusqu'à 2 fois identiques ou différentes par un halogène ou par un radical alkyle ou alkoxy linéaire ou ramifié ayant chacun jusqu'à 6 atomes de carbone,

et leurs sels acceptables du point de vue physiologique,

caractérisé en ce que

au cas ou $R^1$ et $R^2$ ont la définition indiquée ci-dessus mais ne forment pas ensemble un noyau de lactone,

[A] on fait réagir des composés de formule générale (II)

$R^3$-CHO (II)

dans laquelle

$R^3$ à la définition indiquée ci-dessus,

tout d'abord avec des esters acétylacétiques de formule générale (III)

$R^5$-CO-$CH_2$-$CO_2$-$R^4$ (III)

dans laquelle

$R^4$ et $R^5$ ont la défintion indiquée ci-dessus,

le cas échéant avec isolement des composés ylidéniques correspondants de formule générale (IV)

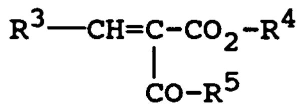
(IV)

dans laquelle

$R^3$, $R^4$ et $R^5$ ont la définition indiquée ci-dessus,

puis

on fait réagir les composés obtenus avec des composés de formule (V)

$R^1$-CO-$CH_2$-$R^2$ (V)

dans laquelle

$R^1$ et $R^2$ ont la définition indiquée ci-dessus,

dans des solvants inertes en présence d'ammoniac ou de sels d'ammonium,

ou directement avec des dérivés de type énamino de formule générale (VI)

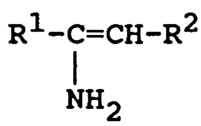
(VI)

dans laquelle

$R^1$ et $R^2$ ont la définition indiquée ci-dessus,

ou bien

[B] on fait réagir les aldéhydes de formule générale (II) tout d'abord avec les composés de formule générale (V), le cas échéant avec isolement du composé ylidéndique de formule générale (VII)

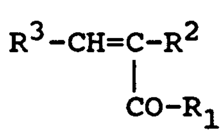
(VII)

dans laquelle

$R^1$, $R^2$ et $R^3$ ont la définition indiquée ci-dessus

et dans une étape suivante, on les fait réagir avec des composés de formule générale (III) indiqués ci-dessus dans des solvants inertes, en présence d'ammoniac ou de sels d'ammonium ou directement avec des dérivés d'acides énamino-carboxyliques de formule générale (VIII)

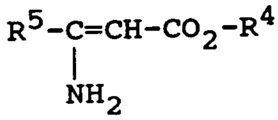

(VIII)

dans laquelle

$R^4$ et $R^5$ ont la définition indiquée ci-dessus,

ou bien au cas où $R^1$ et $R^2$ forment ensemble un noyau de lactone

[C] on prépare tout d'abord par les procédés indiqués en [A] et [B] des composés de formule générale (Ia)

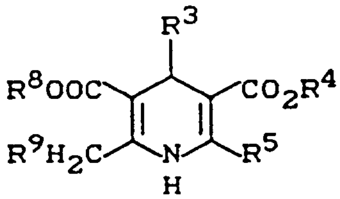

(Ia)

dans laquelle

$R^3$, $R^4$ et $R^5$ ont la définition indiquée ci-dessus,

$R^8$ représente un reste alkyle en $C_1$ à $C_6$

et

$R^9$ est un groupe partant tel que, par exemple, du chlore ou un groupe acétoxy,

et on effectue ensuite par des procédés connus une cyclisation catalysée par un acide ou par une base,

et au cas où $R^4$ ne représente pas de l'hydrogène,

[D] on fait réagir des composés de formule générale (I) dans laquelle $R^1$, $R^2$, $R^3$, $R^4$ et $R^5$ ont la définition indiquée ci-dessus et $R^4$ représente de l'hydrogène, le cas échéant en passant par un dérivé acide réactif, avec les alcools correspondants, et on obtient alors en utilisant l'acide carboxylique de pureté énantiomérique les énantiomères correspondants des esters.